# EUROPEAN PATENT APPLICATION

(11) **EP 3 981 764 A1**
(43) Date of publication of application: **13.04.2022**
(21) Application number: 20818227.9
(22) Date of filing: 02.06.2020
(51) Int. Cl.: C07D 249/20, C07D 401/10, C09K 11/06, H01L 51/50

(54) **COMPOUND HAVING BENZOTRIAZOLE RING STRUCTURE AND ORGANIC ELECTROLUMINESCENCE ELEMENT**

(30) Priority: 06.06.2019 JP 2019106028
(71) Applicant: Hodogaya Chemical Co., Ltd., Tokyo 104-0028 (JP)
(72) Inventor: KASE, Kouki, Tokyo 104-0028 (JP); LIM, Jae-Geon, Tokyo 104-0028 (JP); KIM, Ji-Yung, Tokyo 104-0028 (JP); HIRAYAMA, Yuta, Tokyo 104-0028 (JP); SURUGA, Kazuyuki, Tokyo 104-0028 (JP)
(74) Representative: Forstmeyer, Dietmar
(86) International application number: PCT/JP2020/021845
(87) International publication number: WO 2020/246484

(57) **Abstract**

An objective of the invention is to provide, as a material for highly efficient, highly durable organic EL devices, an organic compound having excellent properties, such as excellent electron injection capability and transportability, hole blocking capability, and high stability in a thin-film state, and also to provide a highly efficient, highly durable organic EL device by using this compound. The present invention has been achieved by focusing on the fact that a benzotriazole ring structure, which has electron affinity, contains nitrogen atoms having the ability to coordinate with a metal and also has excellent heat resistance, and by thus designing and chemically synthesizing various compounds having a benzotriazole ring structure and assessing the properties of various organic EL devices using those compounds, thereby finding out that organic EL devices having excellent properties can be achieved by using specifically-structured compounds having a benzotriazole ring structure.

## Description

### Technical Field

The present invention relates to a compound suitable for an organic electroluminescence device (abbreviated hereinbelow as "organic EL device") which is a self-luminous light-emitting device suitable for various display devices, and to such an organic EL device. More specifically, the present invention relates to a compound having a benzotriazole ring structure, and to an organic EL device employing the compound.

### Background Art

Organic EL devices, which are self-luminous light-emitting devices, are brighter, have better visibility, and are capable of clearer display compared to liquid crystal devices. Therefore, organic EL devices have been actively researched.

In 1987, C. W. Tang et al. of Eastman Kodak Company developed a device having a multilayer structure wherein various roles for light emission were allotted respectively to different materials, thereby achieving practical utilization of organic EL devices using organic materials. They developed a laminate including a layer of a fluorescent substance capable of transporting electrons and a layer of an organic substance capable of transporting holes. Injecting both charges into the fluorescent substance layer causes emission of light, achieving a high luminance of 1,000 cd/m² or higher at a voltage of 10 V or less (see, for example, Patent Literatures 1 and 2).

Many improvements have been heretofore made for practical utilization of organic EL devices. Further subdivision of the various roles within the multilayer structure has yielded an electroluminescent device wherein an anode, a hole injection layer, a hole transport layer, a light-emitting layer, an electron transport layer, an electron injection layer, and a cathode are formed sequentially on a substrate, achieving high efficiency and high durability (see, for example, Non-Patent Literature 1).

To further improve luminous efficiency, attempts have been made to employ triplet excitons. Also, the use of phosphorescent compounds has been investigated (see, for example, Non-Patent Literature 2).

Further, devices employing light emission by thermally activated delayed fluorescence (TADF) have been developed. In 2011, Adachi et al. of Kyushu University achieved an external quantum efficiency of 5.3% with a device using a thermally activated delayed fluorescence material (see, for example, Non-Patent Literature 3).

A light-emitting layer can be prepared by doping a charge-transporting compound, typically called a "host material", with a fluorescent compound, a phosphorescent compound, or a material emitting delayed fluorescence. As described in the aforementioned Non-Patent Literatures, the selection of organic materials in an organic EL device greatly affects various properties such as efficiency and durability of that device (see, for example, Non-Patent Literature 2).

In organic EL devices, charges injected from the respective electrodes recombine in the light-emitting layer to emit light. Thus, what is important is to efficiently deliver the charges, i.e., the holes and electrons, to the light-emitting layer. To achieve this, it is necessary to improve electron injection capability and increase the mobility thereof, to thereby increase the probability of hole-electron recombination within the light-emitting layer. It is also necessary to create an environment that further facilitates recombination, by confining the holes transported from the anode within the light-emitting layer, preventing degradation of the electron transport layer, and also confining excitons generated within the light-emitting layer. By creating such an environment, highly efficient light emission can be achieved. Therefore, the electron-transporting material serves an important role, and there is thus a demand for an electron-transporting material having high electron injection capability, high electron mobility, high hole blocking capability, and high durability against holes.

In terms of device longevity, the material's heat resistance and amorphous properties are also important. With a material having poor heat resistance, thermal decomposition and material degradation occur, even at low temperatures caused by heat produced when the device is driven. With a material having poor amorphous properties, a thin film undergoes crystallization in a short time, resulting in device degradation. Thus, the material to be used requires high heat resistance and good amorphous properties.

A typical light-emitting material is tris(8-hydroxyquinoline) aluminum (abbreviated hereinbelow as Alq3), which is typically used also as an electron-transporting material. Alq3, however, has low electron mobility, and has insufficient hole blocking capability as its work function is 5.6 eV.

Compounds having a benzotriazole structure have been proposed as compounds having improved properties such as electron injection capability and mobility (for example, Patent Literature 3). Devices using such compounds for the electron transport layer are improved in terms of properties such as luminous efficiency, but the improvement is still insufficient, and there is still a demand for an even lower driving voltage and higher luminous efficiency.

Also, 3-(4-biphenylyl)-4-phenyl-5-(4-t-butylphenyl)-1,2,4-triazole (abbreviated hereinbelow as TAZ) has been proposed as an electron-transporting material having excellent hole blocking capability (see, for example, Patent Literature 4).

TAZ has a high work function of 6.6 eV and high hole blocking capability, and is thus used for an electron-transporting hole blocking layer to be layered on the cathode side of a fluorescent light-emitting layer or phosphorescent light-emitting layer produced by vacuum vapor deposition or coating, thus contributing to high efficiency of the organic EL device (see, for example, Non-Patent Literature 4).

Unfortunately, low electron transportability is a major issue for TAZ, and it is necessary to produce an organic EL device by using TAZ in combination with an electron-transporting material having higher electron transportability (see, for example, Non-Patent Literature 5).

Bathocuproine (abbreviated hereinbelow as BCP) has a high work function of 6.7 eV and high hole blocking capability. However, its glass transition point (Tg) is low (83°C). Thus, BCP has poor thin-film stability and cannot be considered as functioning sufficiently as a hole blocking layer.

All of the aforementioned materials lack film stability or have an insufficient hole-blocking function. Thus, in order to improve the properties of organic EL devices, there is a demand for an organic compound having excellent electron injection capability and transportability and hole blocking capability, and also having high stability in a thin-film state.

### Citation List

### Patent Literature

Patent Literature 1: US5792557
Patent Literature 2: US5639914
Patent Literature 3: US9123897
Patent Literature 4: US5869199
Patent Literature 5: US20080027226
Patent Literature 6: EP2684932
Patent Literature 7: US9199966
Patent Literature 8: US6878469

### Non-Patent Literature

Non-Patent Literature 1: Japan Society of Applied Physics, 9th Class, Proceedings, pp. 55-61
(2001) Non-Patent Literature 2: Japan Society of Applied Physics, 9th Class, Proceedings, pp. 23-31
(2001) Non-Patent Literature 3: Appl. Phys. Lett., 98, 083302 (2011)
Non-Patent Literature 4: 50th Meeting of Japan Society of Applied Physics and Related Societies, 28p-A-6 Lecture Proceedings, p. 1413 (2003)
Non-Patent Literature 5: Japan Society of Applied Physics, Journal of Organic Molecules/Bioelectronics Section, Vol. 11, No. 1, pp. 13-19 (2000)

### Summary of Invention

An objective of the present invention is to provide, as a material for highly efficient, highly durable organic EL devices, an organic compound having excellent properties, such as excellent electron injection capability and transportability, hole blocking capability, and high stability in a thin-film state, and also to provide a highly efficient, highly durable organic EL device by using this compound.

Examples of physical properties to be possessed by an organic compound provided by the present invention may include (1) good electron injection capability, (2) high electron mobility, (3) excellent hole blocking capability, (4) stability in a thin-film state, and (5) excellent heat resistance. Examples of physical properties to be possessed by an organic EL device provided by the present invention may include (1) high luminous efficiency and power efficiency, (2) low emission start voltage, (3) low practical driving voltage, and (4) long lifetime.

To achieve the aforementioned objective, Inventors focused on the fact that a benzotriazole ring structure, which has electron affinity, contains nitrogen atoms having the ability to coordinate with a metal, and also has excellent heat resistance. Inventors designed and chemically synthesized various compounds having a benzotriazole ring structure, prototyped various organic EL devices using those compounds, and diligently assessed the properties of those devices, to thus accomplish the present invention.

(1) More specifically, the present invention is a compound having a benzotriazole ring structure, and being represented by general formula (a-1) as below.

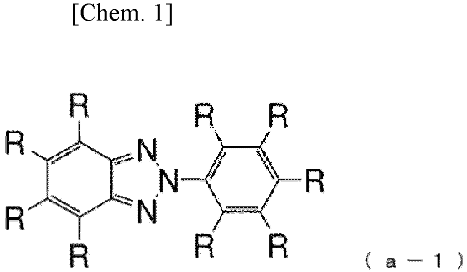

(In the formula, R may be the same or different from one another, and each represents a group represented by structural formula (b-1) as below, a hydrogen atom, a deuterium atom, a fluorine atom, a chlorine atom, a cyano group, a nitro group, a trimethylsilyl group, a triphenylsilyl group, a diphenylphosphinyl group, a diphenylphosphine oxide group, a substituted or unsubstituted aromatic hydrocarbon group, a substituted or unsubstituted aromatic heterocyclic group, a substituted or unsubstituted fused polycyclic aromatic group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, or a cycloalkyloxy group having 5 to 10 carbon atoms and optionally having a substituent, wherein
at least one of Rs is a group represented by the following structural formula (b-1).)

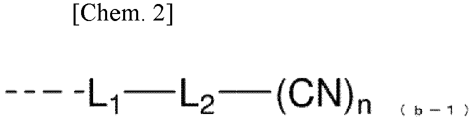

(In the formula, the broken line represents a bonding site,
L₁ represents a single bond, a substituted or unsubstituted aromatic hydrocarbon group, a substituted or unsubstituted aromatic heterocyclic group, or a substituted or unsubstituted fused polycyclic aromatic group,
L₂ represents a substituted or unsubstituted aromatic hydrocarbon group, a substituted or unsubstituted aromatic heterocyclic group, or a substituted or unsubstituted fused polycyclic aromatic group, and
n is an integer of 1 or 2, wherein
L₂ is a trivalent group when n is the integer 2.)

(2) The present invention is also a compound having a benzotriazole ring structure as set forth in clause (1), represented by general formula (a-2) as below.

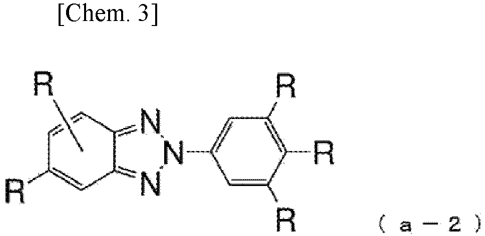

(In the formula, R has the same meaning as in the general formula (a-1).)

(3) The present invention is also a compound having a benzotriazole ring structure as set forth in clause (2), represented by general formula (a-3) as below.

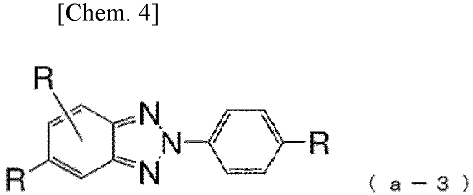

(In the formula, R has the same meaning as in the general formula (a-1).)

(4) The present invention is also a compound having a benzotriazole ring structure as set forth in clause (2), represented by general formula (a-4) as below.

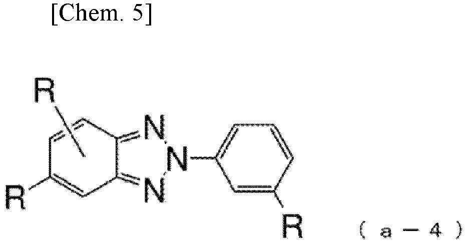

(In the formula, R has the same meaning as in the general formula (a-1).)

(5) The present invention is also a compound having a benzotriazole ring structure as set forth in clause (2), represented by general formula (a-5) as below.

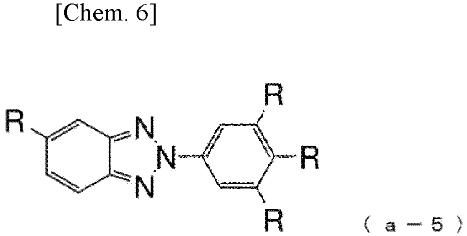

(In the formula, R has the same meaning as in the general formula (a-1).)

(6) The present invention is also a compound having a benzotriazole ring structure as set forth in clause (5), represented by general formula (a-6) as below.

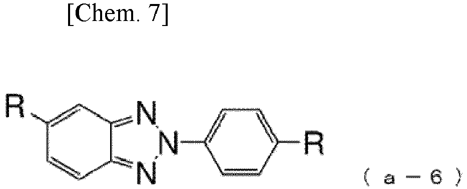

(In the formula, R has the same meaning as in the general formula (a-1).)

(7) The present invention is also a compound having a benzotriazole ring structure as set forth in clause (5), represented by general formula (a-7) as below.

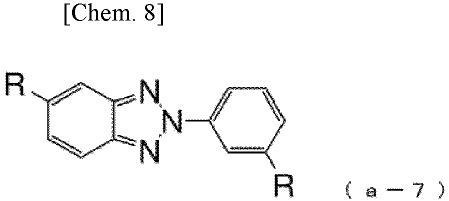

(In the formula, R has the same meaning as in the general formula (a-1).)

(8) The present invention is also a compound having a benzotriazole ring structure as set forth in clauses (1) to (7), wherein n in the structural formula (b-1) is the integer 1.

(9) The present invention is also a compound having a benzotriazole ring structure as set forth in clauses (1) to (7), wherein L₂ in the structural formula (b-1) is a substituted or unsubstituted aromatic hydrocarbon group.

(10) The present invention is also an organic electroluminescence device comprising:
a pair of electrodes; and
at least one organic layer sandwiched between the electrodes, wherein
the at least one organic layer is an organic layer containing the compound having a benzotriazole ring structure as set forth in any one of clauses (1) to (9).

(11) The present invention is also an organic EL device as set forth in clause (10), wherein the organic layer including a benzotriazole ring structure is an electron transport layer.

(12) The present invention is also an organic EL device as set forth in clause (10), wherein the organic layer including a benzotriazole ring structure is a hole blocking layer.

(13) The present invention is also an organic EL device as set forth in clause (10), wherein the organic layer including a benzotriazole ring structure is a light-emitting layer.

(14) The present invention is also an organic EL device as set forth in clause (10), wherein the organic layer including a benzotriazole ring structure is an electron injection layer.

Concrete examples of the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by R in the general formula (a-1) may include a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, an azafluorenyl group, a diazafluorenyl group, an azaspirobifluorenyl group, a diazaspirobifluorenyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, a carbolinyl group, etc., or may be selected from aryl groups having 6 to 30 carbon atoms or hetero aryl groups having 2 to 20 carbon atoms.

Concrete examples of the "substituent" in the "substituted aromatic hydrocarbon group", "substituted aromatic heterocyclic group" or "substituted fused polycyclic aromatic group" as represented by R in the general formula (a-1) may include: a deuterium atom; a cyano group; a nitro group; halogen atoms, such as a fluorine atom, a chlorine atom, a bromine atom, or an iodine atom; silyl groups, such as a trimethylsilyl group, triphenylsilyl group, etc.; linear or branched alkyl groups having 1 to 6 carbon atoms, such as a methyl group, an ethyl group, a propyl group, etc.; linear or branched alkyloxy groups having 1 to 6 carbon atoms, such as a methyloxy group, an ethyloxy group, a propyloxy group, etc.; alkenyl groups, such as a vinyl group, an allyl group, etc.; aryloxy groups, such as a phenyloxy group, a tolyloxy group, etc.; arylalkyloxy groups, such as a benzyloxy group, a phenethyloxy group, etc.; cycloalkyl groups, such as a cyclopentyl group, a cyclohexyl group, a 1-adamantyl group, a 2-adamantyl group, etc.; aromatic hydrocarbon groups or fused polycyclic aromatic groups, such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, etc.; and aromatic heterocyclic groups, such as a pyridyl group, a pyrimidinyl group, a triazinyl group, a thienyl group, a furyl group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a carbolinyl group, etc. These substituents may further be substituted by any of the substituents given as examples above. Further, the aforementioned substituent(s) and the substituted benzene ring, or a plurality of substituents substituting the same benzene ring, may be bonded to each other via a single bond, a substituted or unsubstituted methylene group, an oxygen atom, or a sulfur atom, to form a ring.

Concrete examples of the "linear or branched alkyl group having 1 to 6 carbon atoms", "cycloalkyl group having 5 to 10 carbon atoms" or "linear or branched alkenyl group having 2 to 6 carbon atoms" in the "linear or branched alkyl group having 1 to 6 carbon atoms and optionally having a substituent", "cycloalkyl group having 5 to 10 carbon atoms and optionally having a substituent" or "linear or branched alkenyl group having 2 to 6 carbon atoms and optionally having a substituent" as represented by R in the general formula (a-1) may include a methyl group, an ethyl group, a n-propyl group, an isopropyl group, a n-butyl group, an isobutyl group, a tert-butyl group, a n-pentyl group, an isopentyl group, a neopentyl group, a n-hexyl group, a cyclopentyl group, a cyclohexyl group, a 1-adamantyl group, a 2-adamantyl group, a vinyl group, an allyl group, an isopropenyl group, a 2-butenyl group, etc. The aforementioned substituent(s) and the substituted benzene ring, or a plurality of substituents substituting the same benzene ring, may be bonded to each other via a single bond, a substituted or unsubstituted methylene group, a substituted or unsubstituted amino group, an oxygen atom, or a sulfur atom, to form a ring.

Examples of the "substituent" in the "linear or branched alkyl group having 1 to 6 carbon atoms and optionally having a substituent", "cycloalkyl group having 5 to 10 carbon atoms and optionally having a substituent", or "linear or branched alkenyl group having 2 to 6 carbon atoms and optionally having a substituent" as represented by R in the general formula (a-1) may include the aforementioned examples given for the "substituent" in the "substituted aromatic hydrocarbon group", "substituted aromatic heterocyclic group" or "substituted fused polycyclic aromatic group" as represented by R in the general formula (a-1), and they may take similar forms/configurations as those described above.

Concrete examples of the "linear or branched alkyloxy group having 1 to 6 carbon atoms" or "cycloalkyloxy group having 5 to 10 carbon atoms" in the "linear or branched alkyloxy group having 1 to 6 carbon atoms and optionally having a substituent" or "cycloalkyloxy group having 5 to 10 carbon atoms and optionally having a substituent" as represented by R in the general formula (a-1) may include a methyloxy group, an ethyloxy group, a n-propyloxy group, an isopropyloxy group, a n-butyloxy group, a tert-butyloxy group, a n-pentyloxy group, a n-hexyloxy group, a cyclopentyloxy group, a cyclohexyloxy group, a cycloheptyloxy group, a cyclooctyloxy group, a 1-adamantyloxy group, a 2-adamantyloxy group, etc. The aforementioned substituent(s) and the substituted benzene ring, or a plurality of substituents substituting the same benzene ring, may be bonded to each other via a single bond, a substituted or unsubstituted methylene group, a substituted or unsubstituted amino group, an oxygen atom, or a sulfur atom, to form a ring.

Examples of the "substituent" in the "linear or branched alkyloxy group having 1 to 6 carbon atoms and optionally having a substituent" or "cycloalkyloxy group having 5 to 10 carbon atoms and optionally having a substituent" as represented by R in the general formula (a-1) may include the aforementioned examples given for the "substituent" in the "substituted aromatic hydrocarbon group", "substituted aromatic heterocyclic group" or "substituted fused polycyclic aromatic group" as represented by R in the general formula (a-1), and they may take similar forms/configurations as those described above.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by L₁ and L₂ in the structural formula (b-1) may include the aforementioned examples given for the "aromatic hydrocarbon group", "aromatic heterocyclic group" or "fused polycyclic aromatic group" in the "substituted or unsubstituted aromatic hydrocarbon group", "substituted or unsubstituted aromatic heterocyclic group" or "substituted or unsubstituted fused polycyclic aromatic group" as represented by R in the general formula (a-1), and they may take similar forms/configurations as those described above.

Examples of the "substituent" in the "substituted aromatic hydrocarbon group", "substituted aromatic heterocyclic group" or "substituted fused polycyclic aromatic group" as represented by L₁ and L₂ in the structural formula (b-1) may include the aforementioned examples given for the "substituent" in the "substituted aromatic hydrocarbon group", "substituted aromatic heterocyclic group" or "substituted fused polycyclic aromatic group" as represented by R in the general formula (a-1), and they may take similar forms/configurations as those described above.

The compound having a benzotriazole ring structure represented by the aforementioned general formula (a-1), which is suitably usable for an organic EL device according to the present invention, can be used as a constituent material of an electron injection layer, an electron transport layer, or a hole blocking layer of the organic EL device. The compound having a benzotriazole ring structure represented by the aforementioned general formula (a-1) is a compound having high electron mobility and is thus preferable as a material for an electron injection layer or an electron transport layer.

By using a material for organic EL devices that has excellent properties such as electron injection capability and transportability, thin-film stability and durability, electron-transporting efficiency from the electron transport layer to the light-emitting layer can be improved to thereby improve luminous efficiency, the driving voltage can be reduced and thereby durability can be improved, compared to conventional organic EL devices, and thus, an organic EL device having high efficiency, a low driving voltage, and long lifetime can be achieved.

The compound having a benzotriazole ring structure of the present invention has various properties, such as (1) good electron injection capability, (2) high electron mobility, (3) excellent hole blocking capability, (4) stability in a thin-film state, and (5) excellent heat resistance. The organic EL device of the present invention has various properties, such as (6) high luminous efficiency, (7) a low emission start voltage, (8) a low practical driving voltage, and (9) a long lifetime.

The compound having a benzotriazole ring structure of the present invention has high electron injection capability and mobility. Thus, an organic EL device having an electron injection layer and/or electron transport layer produced by using the present compound as an electron-injecting material and/or electron-transporting material is improved in electron-transporting efficiency to the light-emitting layer and is thus improved in luminous efficiency, and also has a reduced driving voltage and is thereby improved in durability.

The compound having a benzotriazole ring structure of the present invention is characterized by having excellent hole blocking capability and electron transportability, being stable in a thin-film state, and being capable of confining excitons produced in the light-emitting layer. Thus, an organic EL device having a hole blocking layer produced by using the present compound as a hole blocking material has a higher probability of hole-electron recombination, is reduced in thermal inactivation, and thus has high luminous efficiency. Further, the organic EL device is reduced in driving voltage and improved in current resistance, and thereby improved in maximum light emission luminance.

The compound having a benzotriazole ring structure of the present invention has excellent electron transportability and a wide band gap. Thus, in an organic EL device having a light-emitting layer produced using this compound as a host material, by making the light-emitting layer support a dopant, e.g., a fluorescent substance, a phosphorescent substance, or a delayed fluorescent substance, the driving voltage is reduced, and luminous efficiency is improved.

As described above, the compound having a benzotriazole ring structure of the present invention is useful as a material for an electron injection layer, an electron transport layer, a hole blocking layer, or a light-emitting layer of an organic EL device, and can improve luminous efficiency, driving voltage, and durability of conventional organic EL devices.

### Brief Description of Drawings

[Fig. 1] Fig. 1 is a diagram illustrating structures of Compounds 1 to 18 as concrete examples of compounds having a benzotriazole ring structure represented by general formulas (a-1) to (a-7).
[Fig. 2] Fig. 2 is a diagram illustrating structures of Compounds 19 to 33 as concrete examples of compounds having a benzotriazole ring structure represented by general formulas (a-1) to (a-7).
[Fig. 3] Fig. 3 is a diagram illustrating structures of Compounds 34 to 54 as concrete examples of compounds having a benzotriazole ring structure represented by general formulas (a-1) to (a-7).
[Fig. 4] Fig. 4 is a diagram illustrating structures of Compounds 55 to 68 as concrete examples of compounds having a benzotriazole ring structure represented by general formulas (a-1) to (a-7).
[Fig. 5] Fig. 5 is a diagram illustrating structures of Compounds 69 to 82 as concrete examples of compounds having a benzotriazole ring structure represented by general formulas (a-1) to (a-7).
[Fig. 6] Fig. 6 is a diagram illustrating structures of Compounds 83 to 97 as concrete examples of compounds having a benzotriazole ring structure represented by general formulas (a-1) to (a-7).
[Fig. 7] Fig. 7 is a diagram illustrating structures of Compounds 98 to 114 as concrete examples of compounds having a benzotriazole ring structure represented by general formulas (a-1) to (a-7).
[Fig. 8] Fig. 8 is a diagram illustrating structures of Compounds 115 to 130 as concrete examples of compounds having a benzotriazole ring structure represented by general formulas (a-1) to (a-7).
[Fig. 9] Fig. 9 is a diagram illustrating structures of Compounds 131 to 146 as concrete examples of compounds having a benzotriazole ring structure represented by general formulas (a-1) to (a-7).
[Fig. 10] Fig. 10 is a diagram illustrating structures of Compounds 147 to 162 as concrete examples of compounds having a benzotriazole ring structure represented by general formulas (a-1) to (a-7).
[Fig. 11] Fig. 11 is a diagram illustrating structures of Compounds 163 to 179 as concrete examples of compounds having a benzotriazole ring structure represented by general formulas (a-1) to (a-7).
[Fig. 12] Fig. 12 is a diagram illustrating structures of Compounds 180 to 193 as concrete examples of compounds having a benzotriazole ring structure represented by general formulas (a-1) to (a-7).
[Fig. 13] Fig. 13 is a diagram illustrating structures of Compounds 194 to 207 as concrete examples of compounds having a benzotriazole ring structure represented by general formulas (a-1) to (a-7).
[Fig. 14] Fig. 14 is a diagram illustrating a configuration of an organic EL device for Examples 16 to 28 and Comparative Examples 1 and 2.

### Description of Embodiments

The compound having a benzotriazole ring structure of the present invention is a compound represented by one of the general formulas (a-1) to (a-7), but from the viewpoint of electron injection capability and transportability, a compound represented by the general formula (a-3), and more preferably a compound represented by the following general formula (a-3a) or general formula (a-3b), is preferable.

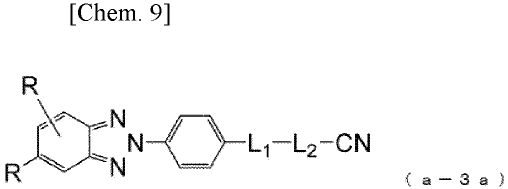

(In the formula, R may be the same or different from one another, and each represents a hydrogen atom, a substituted or unsubstituted aromatic hydrocarbon group, a substituted or unsubstituted aromatic heterocyclic group, or a substituted or unsubstituted fused polycyclic aromatic group, wherein the two Rs are never both hydrogen atoms.
L₁ represents a single bond, a substituted or unsubstituted aromatic hydrocarbon group, or a substituted or unsubstituted fused polycyclic aromatic group, and
L₂ represents a substituted or unsubstituted aromatic hydrocarbon group or a substituted or unsubstituted fused polycyclic aromatic group.)

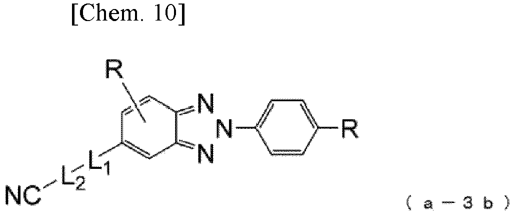

(In the formula, R may be the same or different from one another, and each represents a hydrogen atom, a substituted or unsubstituted aromatic hydrocarbon group, a substituted or unsubstituted aromatic heterocyclic group, or a substituted or unsubstituted fused polycyclic aromatic group, wherein the two Rs are never both hydrogen atoms.
L₁ represents a single bond, a substituted or unsubstituted aromatic hydrocarbon group, or a substituted or unsubstituted fused polycyclic aromatic group, and
L₂ represents a substituted or unsubstituted aromatic hydrocarbon group or a substituted or unsubstituted fused polycyclic aromatic group.)

From the viewpoint of electron injection capability and transportability, it is preferable that L₁ in the general formulas (a-3a) and (a-3b) is a single bond or a 1,4-naphthylene group, and L₂ is a 1,4-phenylene group or a 4,4'-biphenylylene group.

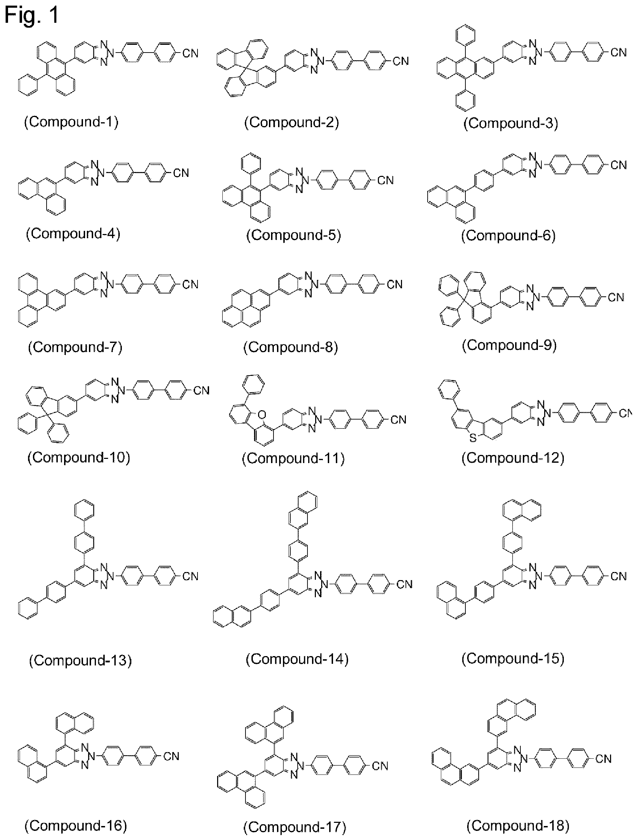

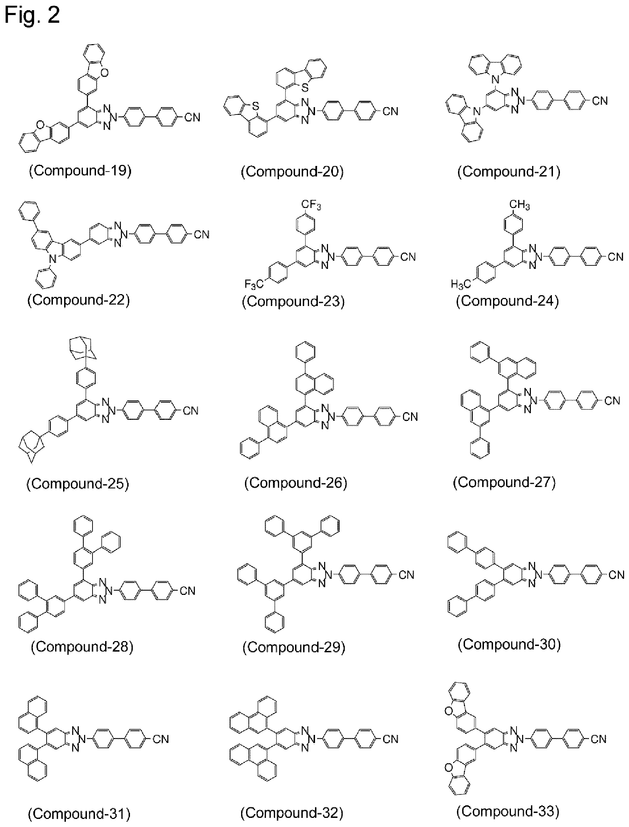

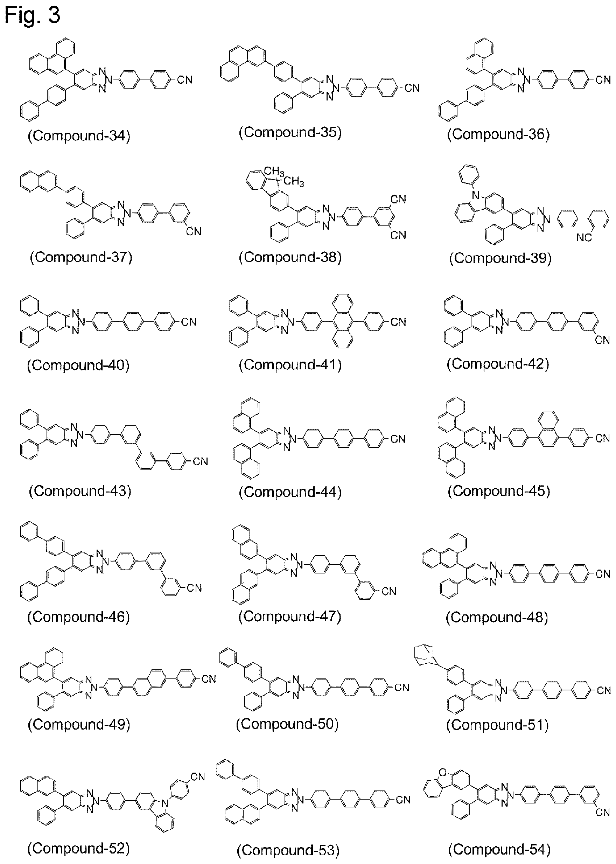

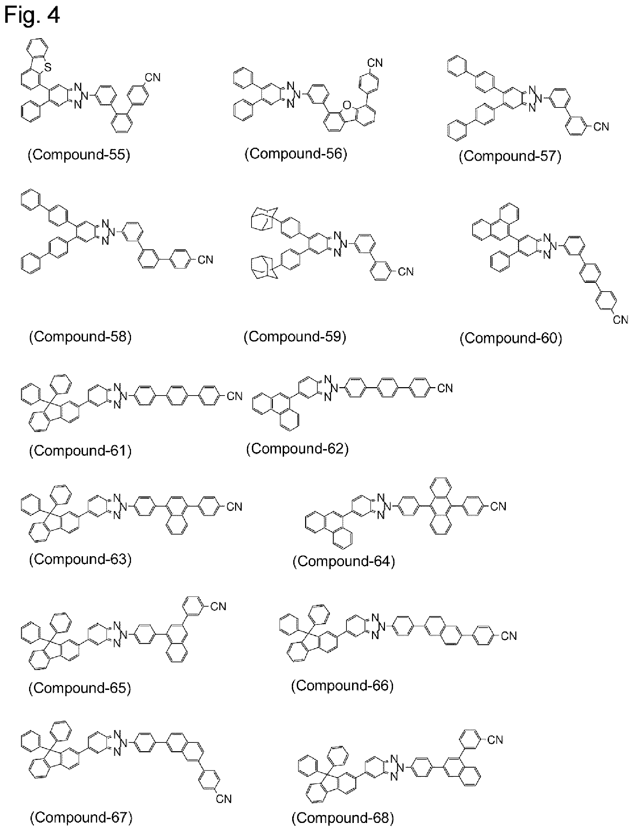

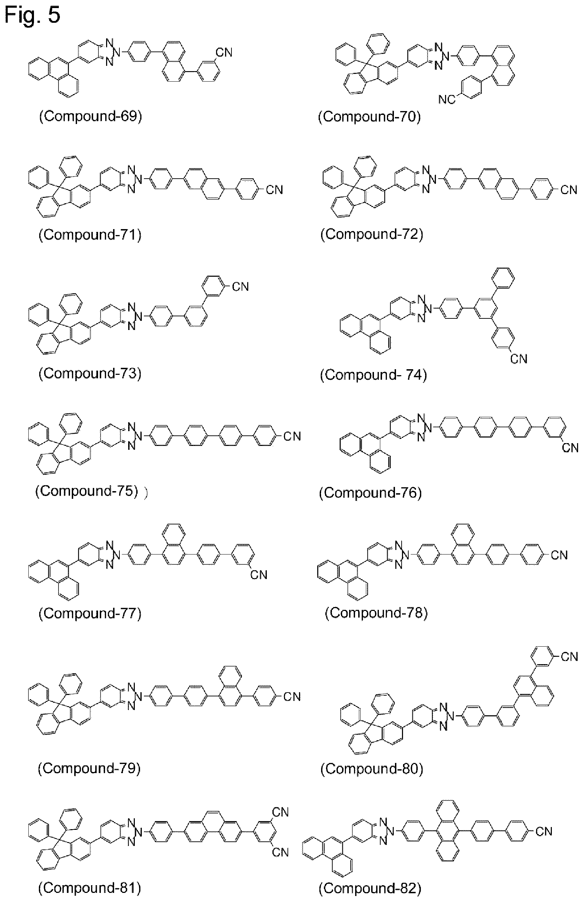

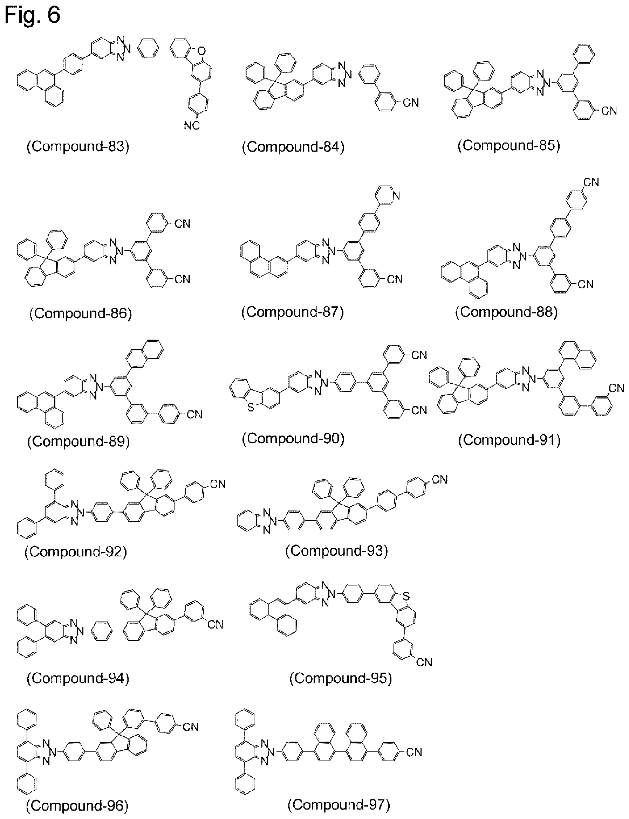

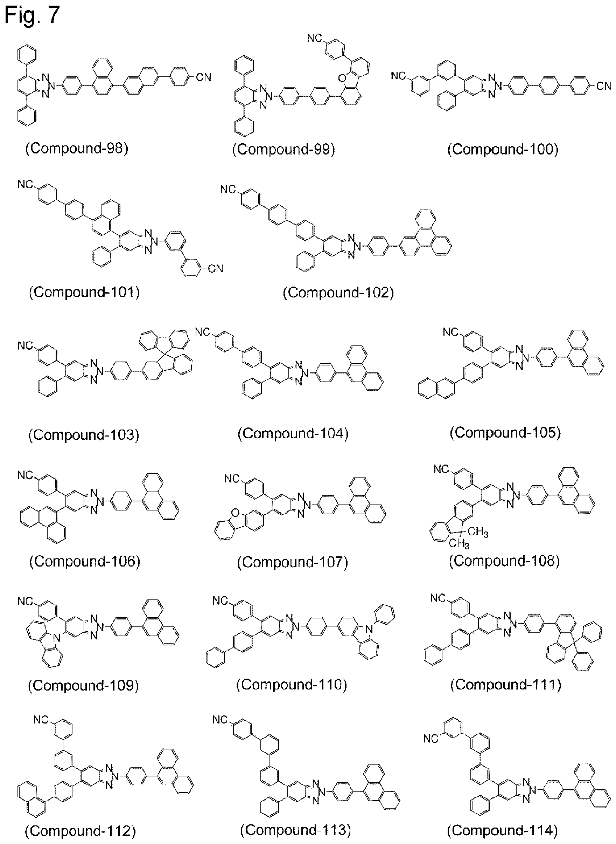

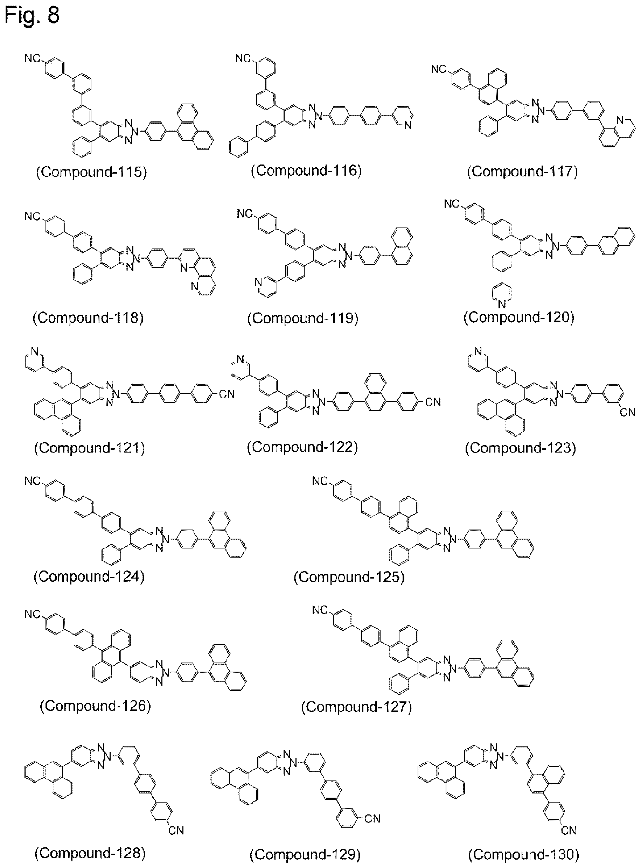

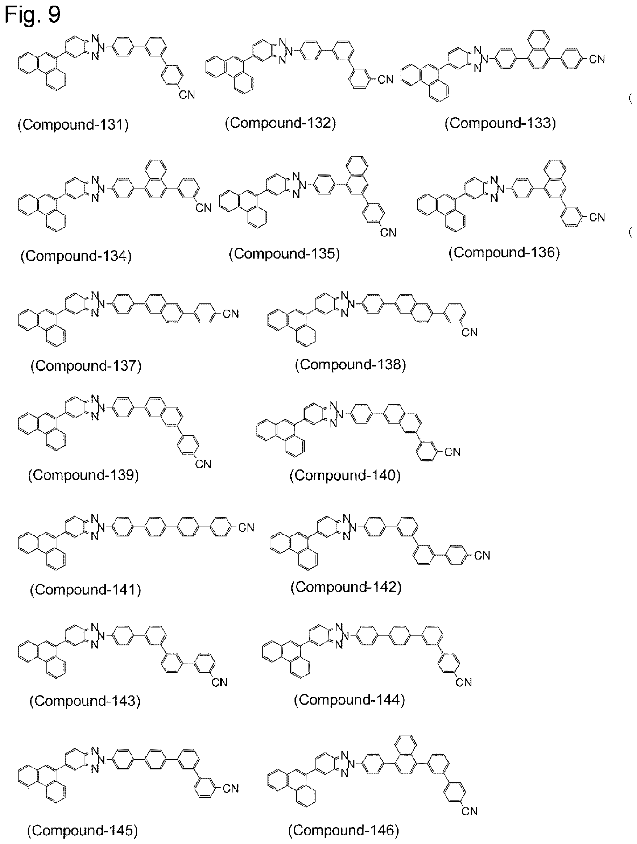

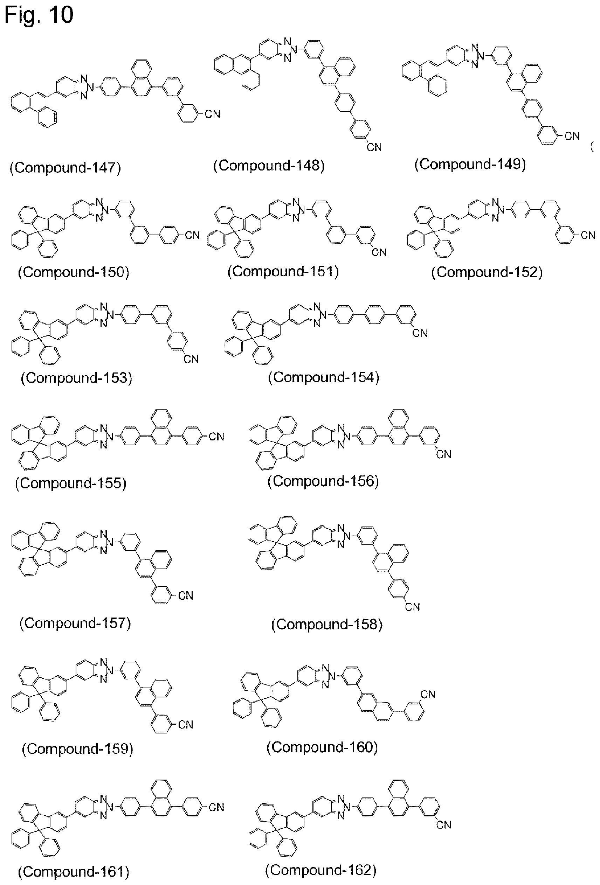

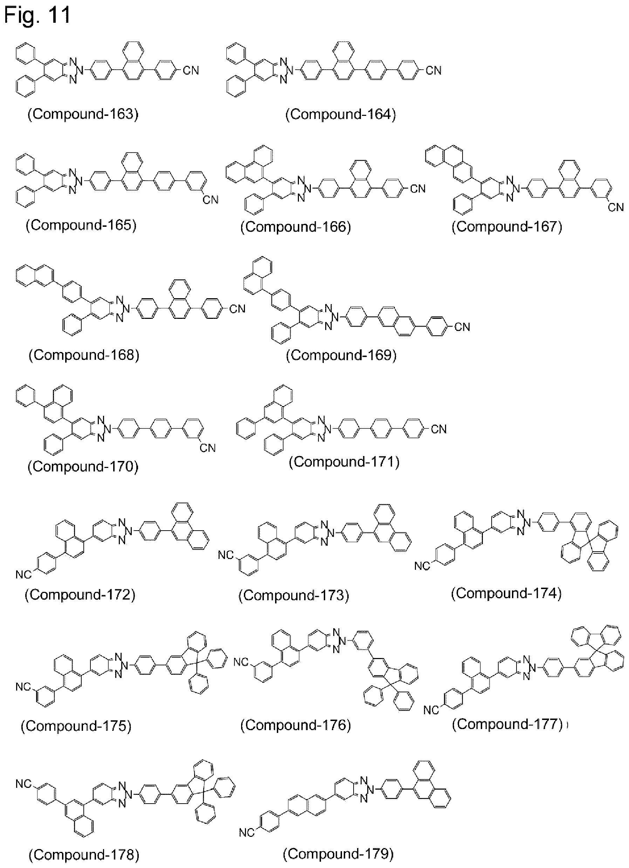

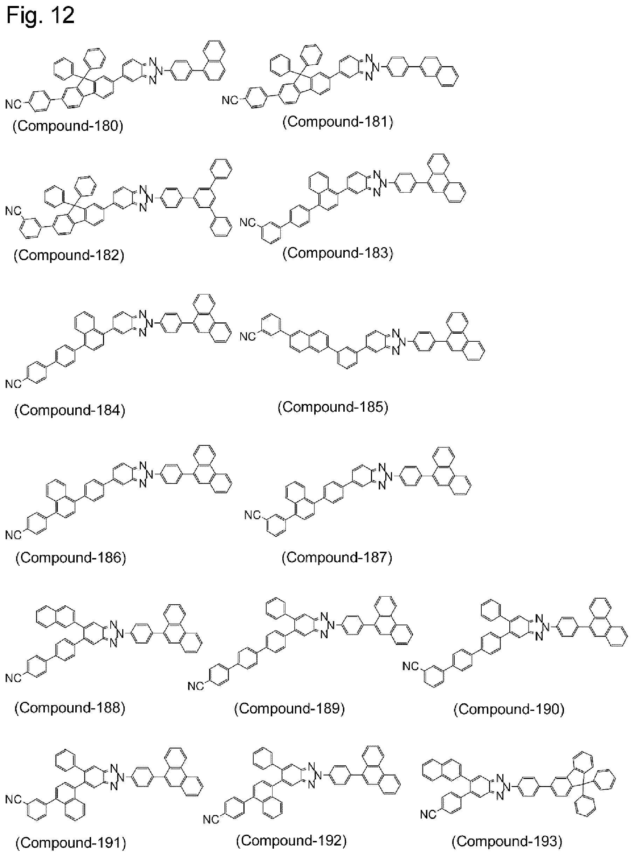

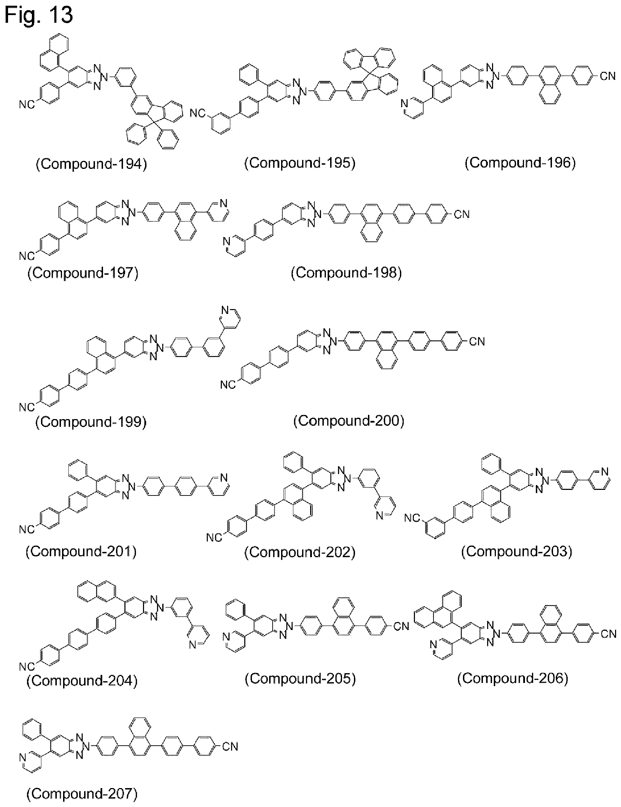

Figs. 1 to 13 illustrate concrete examples of preferred compounds among compounds having a benzotriazole ring structure represented by the general formulas (a-1) to (a-7) that may be suitably used for the organic EL device of the present invention. Note, however, that the compounds are not limited to the illustrated compounds.

The compounds having a benzotriazole ring structure of the present invention are novel compounds. These compounds can be synthesized according to a known method as described below, for example (see, for example, Patent Literatures 3 and 5).

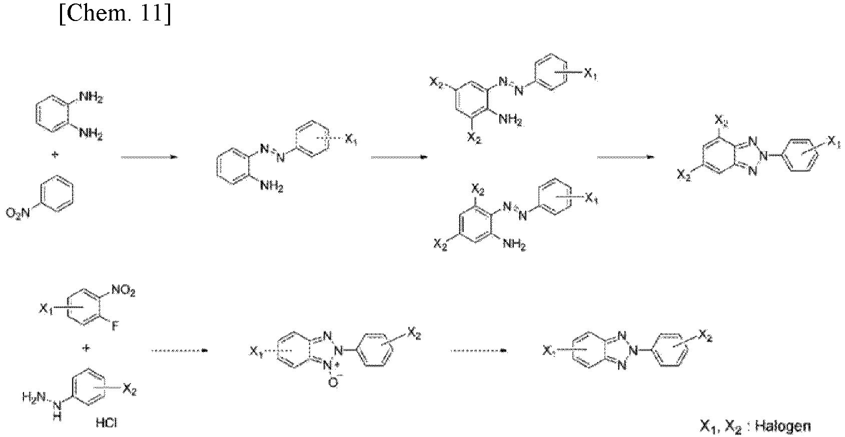

The compound having a benzotriazole ring structure represented by one of general formulas (a-1) to (a-7) can be purified by such techniques as column chromatography purification, adsorption purification with silica gel, activated carbon, activated clay, etc., recrystallization or crystallization using a solvent, sublimation purification, or the like. Compound identification can be achieved by NMR analysis. Physical properties to be measured preferably include such values as the melting point, glass transition point (Tg), work function, or the like. The melting point serves as an index of vapor deposition characteristics. The glass transition point (Tg) serves as an index of stability in a thin-film state. The work function serves as an index of hole transportability and hole blocking capability.

The melting point and glass transition point (Tg) can be measured, for example, with a high-sensitivity differential scanning calorimeter (DSC3100SA from Bruker AXS) using a powder.

The work function can be found, for example, with an ionization potential measurement device (PYS-202 from Sumitomo Heavy Industries, Ltd.) by preparing a 100-nm thin film on an ITO substrate.

A structure of the organic EL device of the present invention may, for example, sequentially include, on a substrate, an anode, a hole injection layer, a hole transport layer, a light-emitting layer, an electron transport layer, an electron injection layer, and a cathode. In other examples, an electron blocking layer may be provided between the hole transport layer and the light-emitting layer, or a hole blocking layer may be provided between the light-emitting layer and the electron transport layer. In such multilayer structures, a single organic layer may have functions of several layers; for example, a single organic layer may have functions of the hole injection layer and the hole transport layer, or functions of the electron injection layer and the electron transport layer. Further, it is possible to stack two or more organic layers having the same function; for example, the structure may include: two stacked hole transport layers; two stacked light-emitting layers; or two stacked electron transport layers.

For the anode in the organic EL device of the present invention, it is possible to use an electrode material having a large work function, such as ITO or gold. For the material for the hole injection layer in the organic EL device of the present invention, it is possible to use: a porphyrin compound typified by copper phthalocyanine; a starburst triphenylamine derivative; an arylamine compound including, in its molecule, two or more triphenylamine structures or carbazolyl structures which are linked by a single bond or a divalent group containing no hetero atom; an acceptor heterocyclic compound such as hexacyanoazatriphenylene; or a coating-type polymer material. These materials can form thin films by known methods, such as vapor deposition, spin coating, ink-jetting, etc.

For the material for the hole transport layer in the organic EL device of the present invention, it is possible to use a benzidine derivative, such as N,N'-diphenyl-N,N'-di(m-tolyl)-benzidine (abbreviated hereinbelow as "TPD"), N,N'-diphenyl-N,N'-di(α-naphthyl)-benzidine (abbreviated hereinbelow as "NPD"), N,N,N',N'-tetrabiphenylylbenzidine, etc., or 1,1-bis[(di-4-tolylamino)phenyl]cyclohexane (abbreviated hereinbelow as "TAPC"), or an arylamine compound including, in its molecule, two or more triphenylamine structures or carbazolyl structures which are linked by a single bond or a divalent group containing no hetero atom.

These materials may each be formed into a film singly, or may be mixed with other materials and formed into a film, and each may be used as a single layer. It is possible to form a laminate structure constituted by layers each formed singly by the respective materials, or a laminate structure constituted by layers formed by mixing a plurality of materials, or a laminate structure constituted by layers each formed singly by the respective materials and layers formed by mixing a plurality of materials.

Further, for the hole injection-transport layer, it is possible to use a coating-type polymer material, such as poly(3,4-ethylenedioxythiophene) (abbreviated hereinbelow as "PEDOT")/poly(styrene sulfonate) (abbreviated hereinbelow as "PSS").

These materials can form thin films by known methods, such as vapor deposition, spin coating, ink-jetting, etc.

For the hole injection layer and the hole transport layer, it is possible to use: a material ordinarily used for such layers and p-doped with trisbromophenylamine hexachloroantimonate or a radialene derivative (see, for example, Patent Literature 6); or a polymer compound having, as a partial structure thereof, a benzidine derivative structure such as TPD.

For the material for the electron blocking layer in the organic EL device of the present invention, it is possible to use a compound having an electron blocking action, such as: a carbazole derivative, such as 4,4',4"-tri(N-carbazolyl)triphenylamine (abbreviated hereinbelow as "TCTA"), 9,9-bis[4-(carbazol-9-yl)phenyl]fluorene, 1,3-bis(carbazol-9-yl)benzene (abbreviated hereinbelow as "mCP"), 2,2-bis(4-carbazol-9-ylphenyl)adamantane (abbreviated hereinbelow as "Ad-Cz"), etc.; or a compound containing a triarylamine structure and a triphenylsilyl group typified by 9-[4-(carbazol-9-yl)phenyl]-9-[4-(triphenylsilyl)phenyl]-9H-fluorene, etc.

These materials may each be formed into a film singly, or may be mixed with other materials and formed into a film, and each may be used as a single layer. It is possible to form a laminate structure constituted by layers each formed singly by the respective materials, or a laminate structure constituted by layers formed by mixing a plurality of materials, or a laminate structure constituted by layers each formed singly by the respective materials and layers formed by mixing a plurality of materials. These materials can form thin films by known methods, such as vapor deposition, spin coating, ink-jetting, etc.

For the material for the light-emitting layer in the organic EL device of the present invention, it is possible to use, other than the compound having a benzotriazole ring structure of the present invention, one of various metal complexes such as a metal complex of a quinolinol derivative, e.g., Alq₃, an anthracene derivative, a bisstyrylbenzene derivative, a pyrene derivative, an oxazole derivative, a poly(para-phenylene vinylene) derivative, etc. The light-emitting layer may be constituted by a host material and a dopant material. For the host material, an anthracene derivative may preferably be used, and also, in addition to such light-emitting materials as the compound having a benzotriazole ring structure of the present invention, it is possible to use, for example, a heterocyclic compound having an indole ring as a partial structure of a fused ring, a heterocyclic compound having a carbazole ring as a partial structure of a fused ring, a carbazole derivative, a thiazole derivative, a benzimidazole derivative, a polydialkylfluorene derivative, etc. For the dopant material, it is possible to use quinacridone, coumarin, rubrene, perylene, a derivative of the above, a benzopyran derivative, a rhodamine derivative, an aminostyryl derivative, etc.

These materials may each be formed into a film singly, or may be mixed with other materials and formed into a film, and each may be used as a single layer. It is possible to form a laminate structure constituted by layers each formed singly by the respective materials, or a laminate structure constituted by layers formed by mixing a plurality of materials, or a laminate structure constituted by layers each formed singly by the respective materials and layers formed by mixing a plurality of materials.

It is also possible to use a phosphorescent substance for the light-emitting material. For the phosphorescent substance, it is possible to use a phosphorescent substance such as a metal complex of iridium, platinum, etc. Examples may include green phosphorescent substances such as Ir(ppy)₃ etc., blue phosphorescent substances such as FIrpic, FIr6, etc., and red phosphorescent substances such as Btp₂Ir(acac) etc. As regards host materials, for the hole injecting/transporting host material, it is possible to use, for example, a carbazole derivative such as 4,4'-di(N-carbazolyl) biphenyl (abbreviated hereinbelow as "CBP"), TCTA, mCP, etc., as well as the compound having a benzotriazole ring structure of the present invention. For the electron-transporting host material, it is possible to use, for example, p-bis(triphenylsilyl)benzene (abbreviated hereinbelow as "UGH2"), 2,2',2"-(1,3,5-phenylene)-tris(1-phenyl-1H-benzimidazole) (abbreviated hereinbelow as "TPBI"), etc.

To avoid concentration quenching, doping of the host material(s) with a phosphorescent light-emitting material is preferably performed by co-vapor deposition within a range of 1 to 30 wt.% with respect to the entire light-emitting layer.

Further, for the light-emitting material, it is possible to use a material emitting delayed fluorescence, e.g., PIC-TRZ, CC2TA, PXZ-TRZ, a CDCB derivative such as 4CzIPN, etc. (see, for example, Non-Patent Literature 3). These materials can form thin films by known methods, such as vapor deposition, spin coating, ink-jetting, etc.

For the material for the hole blocking layer in the organic EL device of the present invention, it is possible to use, other than the compound having a benzotriazole ring structure of the present invention, a compound having a hole blocking action, with examples including phenanthroline derivatives such as BCP, metal complexes of a quinolinol derivative such as BAlq, various rare-earth complexes, oxazole derivatives, triazole derivatives, triazine derivatives, etc. These materials may also serve as materials for the electron transport layer.

These materials may each be formed into a film singly, or may be mixed with other materials and formed into a film, and each may be used as a single layer. It is possible to form a laminate structure constituted by layers each formed singly by the respective materials, or a laminate structure constituted by layers formed by mixing a plurality of materials, or a laminate structure constituted by layers each formed singly by the respective materials and layers formed by mixing a plurality of materials. These materials can form thin films by known methods, such as vapor deposition, spin coating, ink-jetting, etc.

For the material for the electron transport layer in the organic EL device of the present invention, it is possible to use, other than the compound having a benzotriazole ring structure of the present invention, a metal complex of a quinolinol derivative such as Alq₃, BAlq, etc., one of various metal complexes, a triazole derivative, a triazine derivative, an oxadiazole derivative, a pyridine derivative, a benzimidazole derivative, a thiadiazole derivative, an anthracene derivative, a carbodiimide derivative, a quinoxaline derivative, a pyridoindole derivative, a phenanthroline derivative, a silole derivative, etc.

These materials may each be formed into a film singly, or may be mixed with other materials and formed into a film, and each may be used as a single layer. It is possible to form a laminate structure constituted by layers each formed singly by the respective materials, or a laminate structure constituted by layers formed by mixing a plurality of materials, or a laminate structure constituted by layers each formed singly by the respective materials and layers formed by mixing a plurality of materials. These materials can form thin films by known methods, such as vapor deposition, spin coating, ink-jetting, etc.

For the material for the electron injection layer in the organic EL device of the present invention, it is possible to use, other than the compound having a benzotriazole ring structure of the present invention, an alkali metal salt such as lithium fluoride, cesium fluoride, etc., an alkaline-earth metal salt such as magnesium fluoride etc., a metal complex of a quinolinol derivative such as quinolinol lithium etc., a metal oxide such as aluminum oxide etc., or a metal such as ytterbium (Yb), samarium (Sm), calcium (Ca), strontium (Sr), cesium (Cs), etc. The electron injection layer may, however, be omitted by suitable selection of the electron transport layer and the cathode.

For the electron injection layer and the electron transport layer, it is possible to use a material ordinarily used for such layers and n-doped with a metal such as cesium etc.

For the cathode in the organic EL device of the present invention, an electrode material having a low work function, such as aluminum etc., or an alloy having an even lower work function, such as magnesium silver alloy, magnesium indium alloy, aluminum magnesium alloy, etc., may be used as the electrode material.

### Examples

Embodiments of the present invention will be described in further detail below according to working examples. Note, however, that the present invention is not limited to the following examples so long as they do not go beyond the gist of the invention.

### Example 1:

### Synthesis of 2-(4'-cyano-biphenyl-4-yl)-5-(9,9'-spirobi[9H]fluoren-2-yl)-2H-benzotriazole (Compound-2):

A reaction vessel was charged with 9.0 g of 2-(4-chloro-phenyl)-5-(9,9'-spirobi[9H]fluoren-2-yl)-2H-benzotriazole, 2.9 g of 4-cyano-phenylboronic acid, 0.5 g of tris(dibenzylideneacetone) dipalladium(0), 0.2 g of tricyclohexyl phosphine, and 10.5 g of tripotassium phosphate, and the mixture was stirred under reflux overnight in a mixed solvent of 1,4-dioxane and H₂O. After the mixture was allowed to cool, ethyl acetate/H₂O was added into the system, and the organic layer taken out by extraction and liquid separation was concentrated. The obtained concentrate was purified by column chromatography (adsorbent: silica gel; eluent: dichloromethane/n-heptane), to obtain 8.1 g of a yellow powder of 2-(4'-cyano-biphenyl-4-yl)-5-(9,9'-spirobi[9H] fluoren-2-yl)-2H-benzotriazole (Compound-2) (yield: 80%).

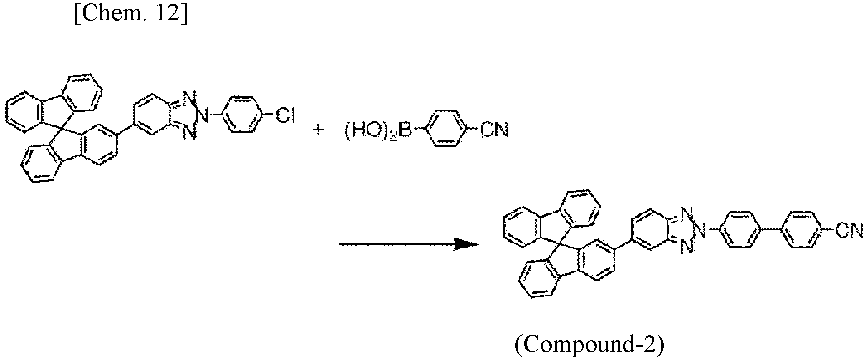

The structure of the obtained yellow powder was identified by NMR

The following 26 hydrogen signals were detected with ¹H-NMR (CDCl₃).
δ (ppm) = 8.46 (2H), 7.97 (2H), 7.94 (1H), 7.90 (3H), 7.81-7.72 (7H), 7.59 (1H), 7.43 (1H), 7.42 (2H), 7.16 (3H), 7.07 (1H), 6.83 (2H), 6.79 (1H).

### Example 2:

### Synthesis of 2-(4-cyano-[1,1',4',1"]terphenyl-4"-yl)-5,6-diphenyl-benzotriazole (Compound-40):

A reaction vessel was charged with 20.0 g of 2-(4-bromophenyl)-5,6-dichloro-benzotriazole, 18.7 g of 4'-(4,4,5,5-tetramethyl-[1,3,2]dioxaborolan-2-yl)biphenyl-4-carbonitrile, 210 mL of toluene, and 70 mL of ethanol, followed by an aqueous solution made by dissolving, in advance, 9.7 g of potassium carbonate in 70 mL of H₂O, and was aerated with nitrogen gas for 30 minutes under ultrasonic irradiation. To the solution aerated with nitrogen gas was added 1.7 g of tetrakistriphenylphosphine palladium, and the mixture was stirred overnight while being heated to reflux. After stirring, the mixture was allowed to cool, and then, methanol and H₂O were added, to perform dispersion washing and filtration and thereby obtain a crude product. The obtained crude product was subjected to dispersion washing with an acetone solvent, to obtain 21.8 g of a golden-yellow powder of 2-(4-cyano-[1,1',4',1"] terphenyl-4"-yl)-5,6-dichloro-benzotriazole (yield: 85%).

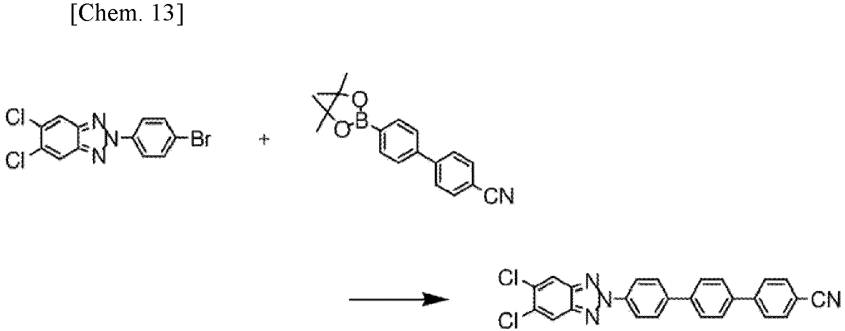

The structure of the obtained golden-yellow powder was identified by NMR.

The following 14 hydrogen signals were detected with ¹H-NMR (CDCl₃).
δ (ppm) = 8.46 (2H), 8.12 (2H), 7.87 (2H), 7.84-7.71 (8H).

Subsequently, 8.0 g of 2-(4-cyano-[1,1',4',1"] terphenyl-4"-yl)-5,6-dichloro-benzotriazole, 6.6 g of phenylboronic acid, 0.8 g of tris(dibenzylideneacetone)dipalladium(0), 1.0 g of tricyclohexylphosphine, and 23.1 g of tripotassium phosphate were charged, and were stirred under reflux overnight in a mixed solvent of 1,4-dioxane and H₂O. After the mixture was allowed to cool, H₂O was added into the system, and a crude product was obtained by dispersion washing and filtration. The obtained crude product was purified by recrystallization with a dichlorobenzene solvent, to obtain 8.3 g of a white powder of 2-(4-cyano-[1,1',4',1"] terphenyl-4"-yl)-5,6-diphenyl-benzotriazole (Compound-40) (yield: 87%).

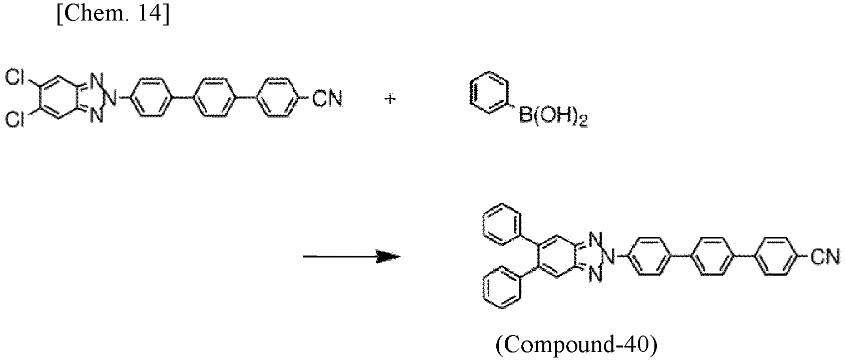

The structure of the obtained white powder was identified by NMR

The following 24 hydrogen signals were detected with ¹H-NMR (CDCl₃).
δ (ppm) = 8.53 (2H), 8.00 (2H), 7.87 (2H), 7.79 (8H), 7.28-7.16 (10H).

### Example 3:

### Synthesis of 2-(4-cyano-[1,1',4',1"]terphenyl-4"-yl)-5,6-di(naphthalen-1-yl)-benzotriazole (Compound-44):

A reaction vessel was charged with 5.0 g of 2-(4-cyano-[1,1',4',1"]terphenyl-4"-yl)-5,6-dichloro-benzotriazole, 6.2 g of 1-naphthaleneboronic acid, 0.5 g of tris(dibenzylideneacetone)dipalladium(0), 0.6 g of tricyclohexylphosphine, and 14.4 g of tripotassium phosphate, and the mixture was stirred under reflux overnight in a mixed solvent of 1,4-dioxane and H₂O. After the mixture was allowed to cool, H₂O was added into the system, and a crude product was obtained by dispersion washing and filtration. The obtained crude product was purified by crystallization with a dichlorobenzene/acetone mixed solvent, to obtain 4.6 g of a white powder of 2-(4-cyano-[1,1',4',1"]terphenyl-4"-yl)-5,6-di(naphthalen-1-yl)-benzotriazole (Compound-44) (yield: 65%).

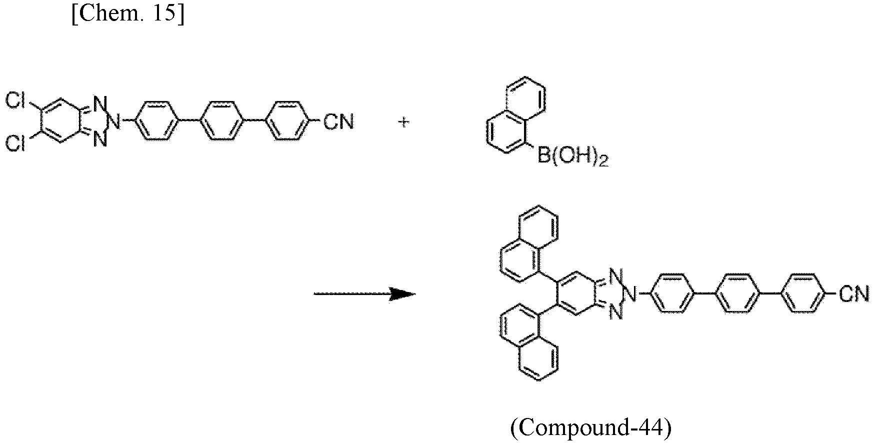

The structure of the obtained white powder was identified by NMR

The following 28 hydrogen signals were detected with ¹H-NMR (CDCl₃).
δ (ppm) = 8.58 (2H), 8.14 (2H), 7.94-7.73 (13H), 7.60 (3H), 7.46 (2H), 7.28 (3H), 7.11 (1H), 7.01 (2H).

### Example 4:

### Synthesis of 2-(4"-cyano-[1,1';4',1"]terphenyl-4-yl)-5-(phenanthren-9-yl)-6-phenyl-2H-benzotriazole (Compound-48):

A reaction vessel was charged with 13.1 g of 5-chloro-2-(4"-cyano-[1,1';4',1"]terphenyl-4-yl)-6-phenyl-2H-benzotriazole, 12.6 g of 9-phenanthreneboronic acid, 1.5 g of tris(dibenzylideneacetone)dipalladium(0), 1.5 g of tricyclohexylphosphine, and 23.0 g of tripotassium phosphate, and the mixture was stirred under reflux overnight in a mixed solvent of 1,4-dioxane and H₂O. After the mixture was allowed to cool, methanol was added into the system, and a crude product was obtained by dispersion washing and filtration. The obtained crude product was purified by recrystallization with a chlorobenzene solvent, to obtain 6.6 g of a white powder of 2-(4"-cyano-[1,1';4',1"] terphenyl-4-yl)-5-(phenanthren-9-yl)-6-phenyl-2H-benzotriazole (Compound-48) (yield: 39%).

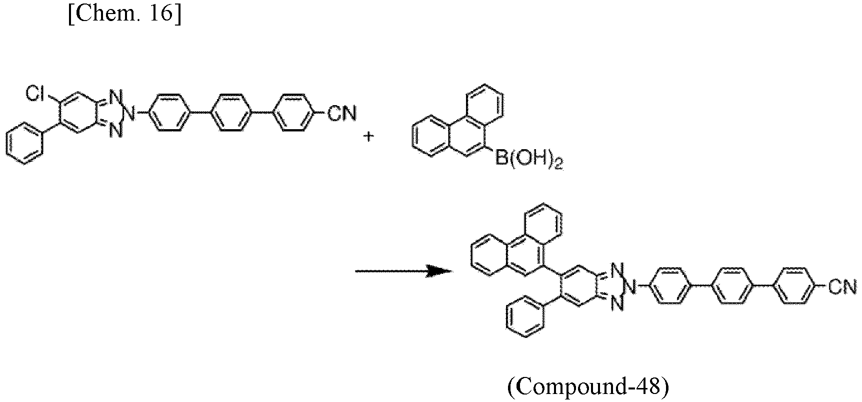

The structure of the obtained white powder was identified by NMR

The following 28 hydrogen signals were detected with ¹H-NMR (CDCl₃).
δ (ppm) = 8.68 (2H), 8.56 (2H), 8.10 (2H), 7.89 (2H), 7.84 (3H), 7.77 (6H), 7.70 (1H), 7.67 (2H), 7.59 (2H), 7.42 (1H), 7.23 (2H), 7.00 (3H).

### Example 5:

### Synthesis of 2-[4-{4-(4'-cyano-biphenyl-4-yl)-naphthalen-1-yl}-phenyl]-5-(phenanthren-9-yl)-2H-benzotri azole (Compound-78):

A reaction vessel was charged with 5.6 g of 2-(4-chloro-phenyl)-5-(phenanthren-9-yl)-2H-benzotriazole, 6.2 g of 4'-{4-(4,4,5,5-tetramethyl-[1,3,2]dioxaborolan-2-yl)-naphthalen-1-yl}-biphenyl-4-carbonitrile, 0.4 g of tris(dibenzylideneacetone)dipalladium(0), 0.4 g of tricyclohexylphosphine, and 5.9 g of tripotassium phosphate, and the mixture was stirred under reflux overnight in a mixed solvent of 1,4-dioxane and H₂O. After the mixture was allowed to cool, methanol was added into the system, and a crude product was obtained by dispersion washing and filtration. The obtained crude product was purified by crystallization with a chlorobenzene/acetone mixed solvent, to obtain 3.3 g of a pale-yellow powder of 2-[4-{4-(4'-cyano-biphenyl-4-yl)-naphthalen-1-yl}-phenyl]-5-(phenanthren-9-yl)-2H-benzotri azole (Compound-78) (yield: 35%).

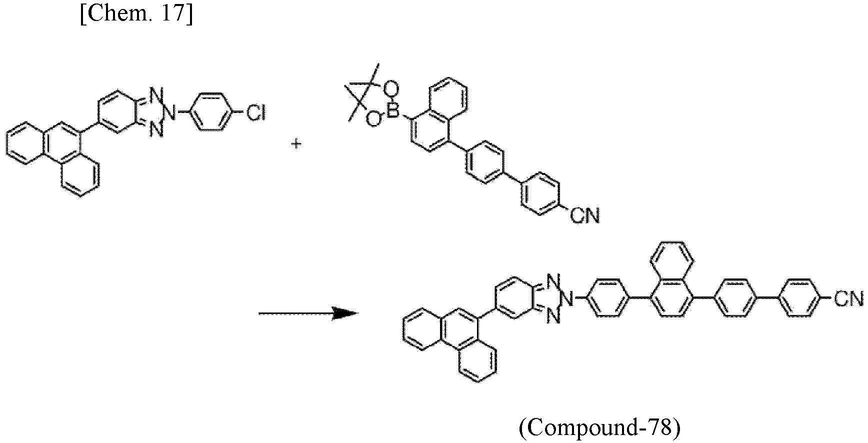

The structure of the obtained pale-yellow powder was identified by NMR.

The following 30 hydrogen signals were detected with ¹H-NMR (CDCl₃).
δ (ppm) = 8.86 (1H), 8.80 (1H), 8.60 (2H), 8.18 (1H), 8.10 (2H), 8.08 (1H), 8.00 (1H), 7.97 (1H), 7.88-7.65 (15H), 7.61 (3H), 7.55 (2H).

### Example 6:

### Synthesis of 2-(4-(phenanthren-9-yl)-phenyl)-5-(4'-cyano-biphenyl-4-yl)-6-phenyl-benzotriazole (Compound-104):

A reaction vessel was charged with 26.0 g of 2-(4-bromophenyl)-5-chloro-6-phenyl-benzotriazole, 15.8 g of 9-phenanthreneboronic acid, 180 mL of toluene, and 45 mL of ethanol, followed by an aqueous solution made by dissolving, in advance, 11.2 g of potassium carbonate in 40 mL of H₂O, and was aerated with nitrogen gas for 30 minutes under ultrasonic irradiation. To the solution aerated with nitrogen gas was added 1.6 g of tetrakistriphenylphosphine palladium, and the mixture was stirred overnight while being heated to reflux. After stirring, the mixture was allowed to cool, and then, ethyl acetate and H₂O were added, and the organic layer was taken out by extraction and liquid separation, and a crude product was obtained by concentration under reduced pressure. The obtained crude product was purified by crystallization with an acetone/methanol mixed solvent, to obtain 28.0 g of a yellow powder of 2-(4-(phenanthren-9-yl)-phenyl)-5-chloro-6-phenyl-benzotriazole (yield: 86%).

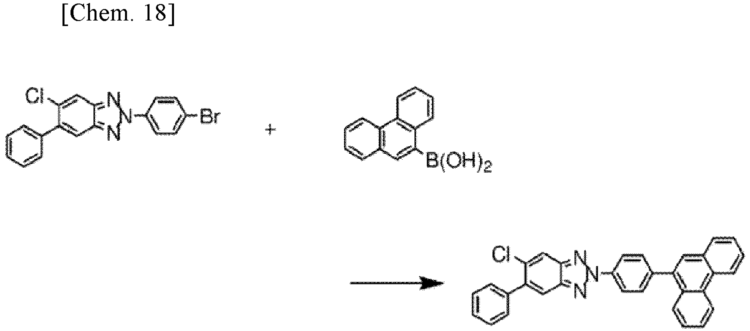

The structure of the obtained yellow powder was identified by NMR

The following 20 hydrogen signals were detected with ¹H-NMR (CDCl₃).
δ (ppm) = 8.84 (1H), 8.78 (1H), 8.54 (2H), 8.15 (1H), 7.97 (3H), 7.84-7.45 (12H).

Subsequently, 14.0 g of 2-(4-(phenanthren-9-yl)-phenyl)-5-chloro-6-phenyl-benzotriazole, 9.3 g of 4'-(4,4,5,5-tetramethyl-[1,3,2]dioxaborolan-2-yl)biphenyl-4-carbonitrile, 0.8 g of tris(dibenzylideneacetone)dipalladium(0), 0.8 g of tricyclohexylphosphine, and 12.3 g of tripotassium phosphate were charged, and were stirred under reflux overnight in a mixed solvent of 1,4-dioxane and H₂O. After the mixture was allowed to cool, methanol was added into the system, and the precipitated solid was filtered, to obtain a crude product. The obtained crude product was purified by recrystallization with a monochlorobenzene solvent, to obtain 11.8 g of a white powder of 2-(4-(phenanthren-9-yl)-phenyl)-5-(4'-cyano-biphenyl-4-yl)-6-phenyl-benzotriazole (Compound-104) (yield: 65%).

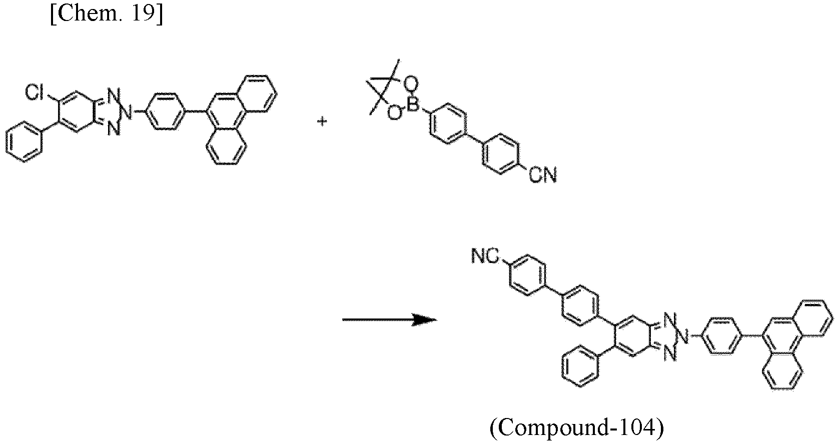

The structure of the obtained white powder was identified by NMR.

The following 28 hydrogen signals were detected with ¹H-NMR (CDCl₃).
δ (ppm) = 8.85 (1H), 8.78 (1H), 8.58 (2H), 8.07 (2H), 7.98 (2H), 7.87-7.64 (9H), 7.61 (1H), 7.53 (2H), 7.40-7.23 (8H).

### Example 7:

### Synthesis of 2-(4‴-cyano-[1,1';4',1";4",1‴]quaterphenyl-4-yl)-5-(phenanthren-9-yl)-2H-benzotriazole (Compound-141):

A reaction vessel was charged with 10.0 g of 2-(4-chloro-phenyl)-5-(phenanthren-9-yl)-2H-benzotriazole, 9.9 g of 4-(4,4,5,5-tetramethyl-[1,3,2]dioxaborolan-2-yl)-[1,1';4',1"]terphenyl-4"-carbonitrile, 0.7 g of tris(dibenzylideneacetone)dipalladium(0), 0.7 g of tricyclohexylphosphine, and 10.5 g of tripotassium phosphate, and the mixture was stirred under reflux overnight in a mixed solvent of 1,4-dioxane and H₂O. After the mixture was allowed to cool, methanol was added into the system, and a crude product was obtained by dispersion washing and filtration. The obtained crude product was purified by recrystallization with a 1,2-dichlorobenzene solvent, to obtain 10.4 g of a yellow powder of 2-(4‴-cyano-[1,1';4',1";4",1‴] quaterphenyl-4-yl)-5-(phenanthren-9-yl)-2H-benzotriazole (Compound-141) (yield: 67%).

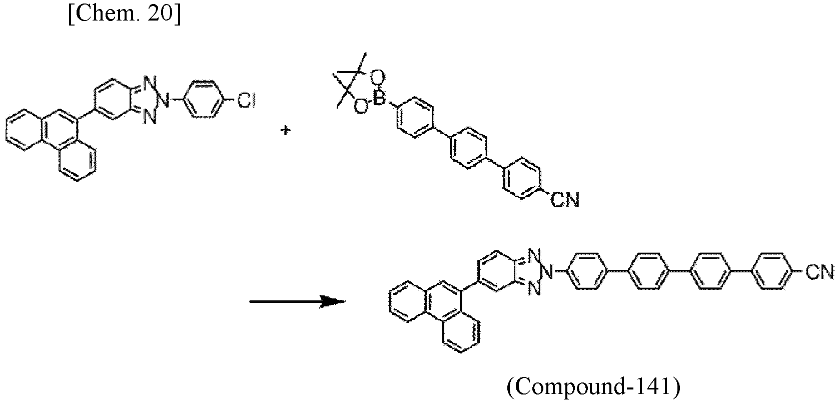

The structure of the obtained yellow powder was identified by NMR

The following 30 hydrogen signals were detected with ¹H-NMR (CDCl₃).
δ (ppm) = 8.82 (1H), 8.77 (1H), 8.53 (2H), 8.13 (1H), 8.07 (1H), 7.95 (2H), 7.88 (2H), 7.81 (7H), 7.77 (5H), 7.75-7.60 (7H), 7.57 (1H).

### Example 8:

### Synthesis of 2-[4-{4-(4-cyano-phenyl)-naphthalen-1-yl}-phenyl]-5-(9,9'-spirobi[9H]fluoren-2-yl)-2H-ben zotriazole (Compound-155):

A reaction vessel was charged with 9.0 g of 2-(4-chloro-phenyl)-5-(9,9'-spirobi[9H]fluoren-2-yl)-2H-benzotriazole, 6.5 g of 4-{4-(4,4,5,5-tetramethyl-[1,3,2]dioxaborolan-2-yl)-naphthalen-1-yl}-phenyl-carbonitrile, 0.3 g of tris(dibenzylideneacetone)dipalladium(0), 0.2 g of tricyclohexylphosphine, and 7.0 g of tripotassium phosphate, and the mixture was stirred under reflux overnight in a mixed solvent of 1,4-dioxane and H₂O. After the mixture was allowed to cool, ethyl acetate/H₂O was added into the system, and the organic layer taken out by extraction and liquid separation was concentrated. The obtained crude product was purified by recrystallization with an acetone solvent, to obtain 7.6 g of a white powder of 2-[4-{4-(4-cyano-phenyl)-naphthalen-1-yl}-phenyl]-5-(9,9'-spirobi[9H]fluoren-2-yl)-2H-ben zotriazole (Compound-155) (yield: 62%).

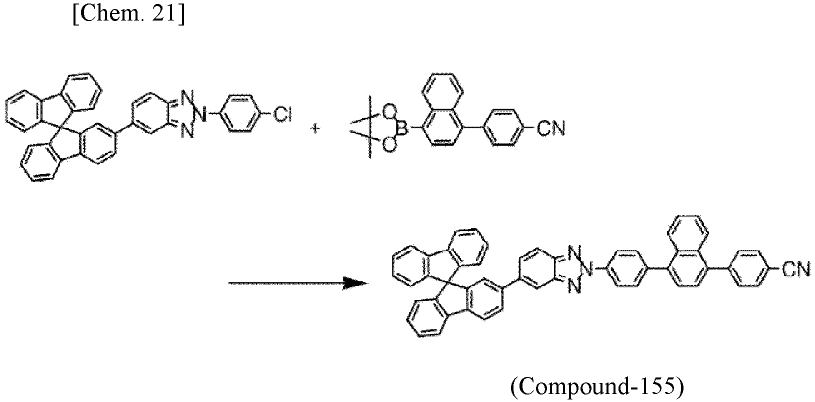

The structure of the obtained white powder was identified by NMR.

The following 32 hydrogen signals were detected with ¹H-NMR (CDCl₃).
δ (ppm) = 8.50 (2H), 8.03 (1H), 8.00 (1H), 7.98 (1H), 7.91 (5H), 7.85 (2H), 7.80-7.65 (5H), 7.63-7.38 (8H), 7.17 (3H), 7.09 (1H), 6.84 (2H), 6.80 (1H).

### Example 9:

### Synthesis of 5,6-diphenyl-2-[4-{4-(4'-cyano-biphenyl-4-yl)-naphthalen-1-yl}-phenyl]-2H-benzotriazole (Compound-164):

A reaction vessel was charged with 12.0 g of 5,6-dichloro-2-[4-{4-(4'-cyano-biphenyl-4-yl)-naphthalen-1-yl}-phenyl]-2H-benzotriazole, 7.7 g of phenylboronic acid, 1.0 g of tris(dibenzylideneacetone)dipalladium(0), 1.2 g of tricyclohexylphosphine, and 26.9 g of tripotassium phosphate, and the mixture was stirred under reflux overnight in a mixed solvent of 1,4-dioxane and H₂O. After the mixture was allowed to cool, H₂O was added into the system, and a crude product was obtained by dispersion washing and filtration. The obtained crude product was purified by recrystallization with a chlorobenzene solvent, to obtain 8.2 g of a white powder of 5,6-diphenyl-2-[4-{4-(4'-cyano-biphenyl-4-yl)-naphthalen-1-yl}-phenyl]-2H-benzotriazole (Compound-164) (yield: 60%).

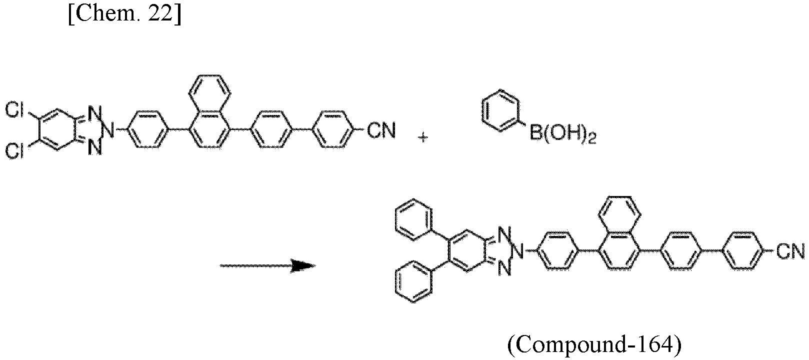

The structure of the obtained white powder was identified by NMR.

The following 30 hydrogen signals were detected with ¹H-NMR (CDCl₃).
δ (ppm) = 8.55 (2H), 8.04 (2H), 8.00 (2H), 7.80 (4H), 7.77 (4H), 7.69 (2H), 7.57 (2H), 7.52 (2H), 7.29-7.16 (10H).

### Example 10:

### Synthesis of 5-{4-(4-cyano-phenyl)-naphthalen-1-yl}-2-{4-(phenanthren-9-yl)-phenyl}-2H-benzotriazole (Compound-172):

A reaction vessel was charged with 11.0 g of 5-chloro-2-{4-(phenanthren-9-yl)-phenyl}-2H-benzotriazole, 10.1 g of 4-{4-(4,4,5,5-tetramethyl-[1,3,2]dioxaborolan-2-yl)-naphthalen-1-yl}-phenyl-carbonitrile, 0.7 g of tris(dibenzylideneacetone)dipalladium(0), 0.8 g of tricyclohexylphosphine, and 11.5 g of tripotassium phosphate, and the mixture was stirred under reflux overnight in a mixed solvent of 1,4-dioxane and H₂O. After the mixture was allowed to cool, methanol was added into the system, and a crude product was obtained by dispersion washing and filtration. The obtained crude product was purified by recrystallization with a toluene solvent, to obtain 10.5 g of a pale-yellow powder of 5-{4-(4-cyano-phenyl)-naphthalen-1-yl}-2-{4-(phenanthren-9-yl)-phenyl}-2H-benzotriazole (Compound-172) (yield: 71%).

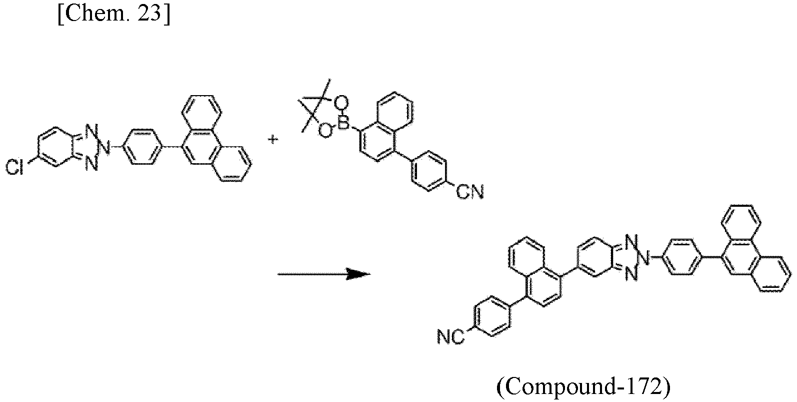

The structure of the obtained pale-yellow powder was identified by NMR.

The following 26 hydrogen signals were detected with ¹H-NMR (CDCl₃).
δ (ppm) = 8.82 (1H), 8.80 (1H), 8.57 (2H), 8.12 (1H), 8.11 (1H), 8.04 (1H), 7.98 (1H), 7.94 (1H), 7.88 (1H), 7.84 (2H), 7.79 (2H), 7.78 (1H), 7.74-7.56 (8H), 7.53 (1H), 7.51 (1H), 7.50 (1H).

### Example 11:

### Synthesis of 5-{4-(4'-cyano-biphenyl-4-yl)-naphthalen-1-yl}-2-{4-(phenanthren-9-yl)-phenyl}-2H-benzotr iazole (Compound-184):

A reaction vessel was charged with 10.0 g of 5-chloro-2-{4-(phenanthren-9-yl)-phenyl}-2H-benzotriazole, 11.2 g of 4'-{4-(4,4,5,5-tetramethyl-[1,3,2]dioxaborolan-2-yl)-naphthalen-1-yl}-biphenyl-4-carbonitrile, 0.7 g of tris(dibenzylideneacetone)dipalladium(0), 0.7 g of tricyclohexylphosphine, and 10.5 g of tripotassium phosphate, and the mixture was stirred under reflux overnight in a mixed solvent of 1,4-dioxane and H₂O. After the mixture was allowed to cool, methanol was added into the system, and a crude product was obtained by dispersion washing and filtration. The obtained crude product was purified by recrystallization with a chlorobenzene solvent, to obtain 4.1 g of a pale-yellow powder of 5-{4-(4'-cyano-biphenyl-4-yl)-naphthalen-1-yl}-2-{4-(phenanthren-9-yl)-phenyl}-2H-benzotr iazole (Compound-184) (yield: 25%).

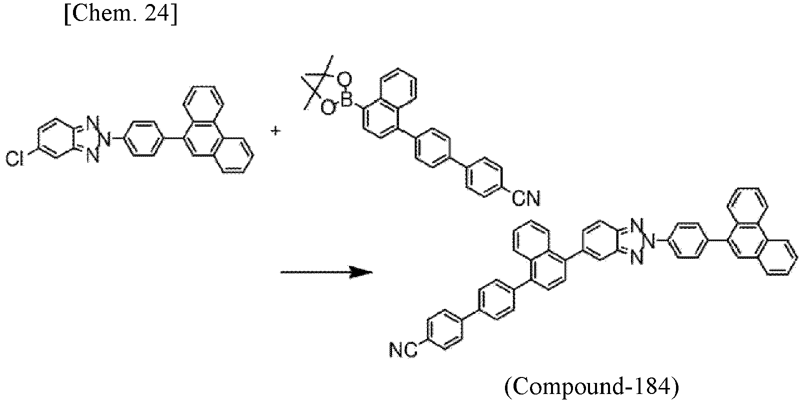

The structure of the obtained pale-yellow powder was identified by NMR

The following 30 hydrogen signals were detected with ¹H-NMR (CDCl₃).
δ (ppm) = 8.82 (1H), 8.76 (1H), 8.57 (2H), 8.13 (1H), 8.10 (1H), 8.05 (2H), 7.98 (1H), 7.94 (1H), 7.84-7.75 (9H), 7.74-7.54 (9H), 7.51 (2H).

### Example 12:

### Synthesis of 5-(4"-cyano-[1,1';4',1"]terphenyl-4-yl)-2-{4-(phenanthren-9-yl)-phenyl}-6-phenyl-2H-benzot riazole (Compound-189):

A reaction vessel was charged with 13.0 g of 5-chloro-2-{4-(phenanthren-9-yl)-phenyl}-6-phenyl-2H-benzotriazole, 8.6 g of 4-(4,4,5,5-tetramethyl-[1,3,2]dioxaborolan-2-yl)-[1,1';4',1"]terphenyl-4"-carbonitrile, 0.7 g of tris(dibenzylideneacetone)dipalladium(0), 0.8 g of tricyclohexylphosphine, and 11.5 g of tripotassium phosphate, and the mixture was stirred under reflux overnight in a mixed solvent of 1,4-dioxane and H₂O. After the mixture was allowed to cool, methanol was added into the system, and a crude product was obtained by dispersion washing and filtration. The obtained crude product was purified by recrystallization with a chlorobenzene solvent, to obtain 10.0 g of a white powder of 5-(4"-cyano-[1,1';4',1"] terphenyl-4-yl)-2-{4-(phenanthren-9-yl)-phenyl}-6-phenyl-2H-benzotriazole (Compound-189) (yield: 53%).

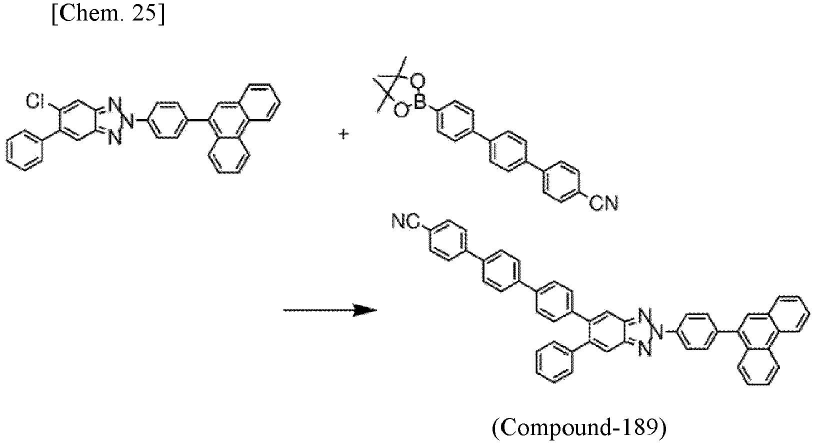

The structure of the obtained white powder was identified by NMR

The following 32 hydrogen signals were detected with ¹H-NMR (CDCl₃).
δ (ppm) = 8.85 (1H), 8.78 (1H), 8.58 (2H), 8.07 (2H), 7.99 (2H), 7.85-7.61 (14H), 7.58 (2H), 7.32 (8H).

### Example 13:

### Synthesis of 5-(4'-cyano-biphenyl-4-yl)-6-phenyl-2-(4'-pyridine-3-yl-biphenyl-4-yl)-2H-benzotriazole (Compound-201):

A reaction vessel was charged with 12.0 g of 5-chloro-6-phenyl-2-(4'-pyridine-3-yl-biphenyl-4-yl)-2H-benzotriazole, 8.4 g of 4'-(4,4,5,5-tetramethyl-[1,3,2]dioxaborolan-2-yl)biphenyl-4-carbonitrile, 0.7 g of tris(dibenzylideneacetone)dipalladium(0), 0.7 g of tricyclohexylphosphine, and 11.1 g of tripotassium phosphate, and the mixture was stirred under reflux overnight in a mixed solvent of 1,4-dioxane and H₂O. After the mixture was allowed to cool, methanol was added into the system, and a crude product was obtained by dispersion washing and filtration. The obtained crude product was purified by recrystallization with a chlorobenzene solvent, to obtain 6.4 g of a white powder of 5-(4'-cyano-biphenyl-4-yl)-6-phenyl-2-(4'-pyridine-3-yl-biphenyl-4-yl)-2H-benzotriazole (Compound-201) (yield: 41%).

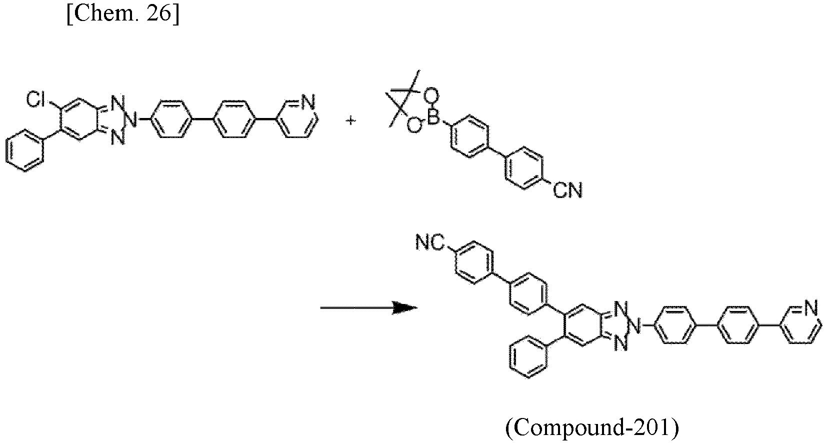

The structure of the obtained white powder was identified by NMR.

The following 27 hydrogen signals were detected with ¹H-NMR (CDCl₃).
δ (ppm) = 8.46 (1H), 8.66 (1H), 8.53 (2H), 8.04 (2H), 7.98 (1H), 7.89 (2H), 7.84 (2H), 7.75 (6H), 7.52 (2H), 7.44 (1H), 7.37-7.22 (7H).

### Example 14:

Using a high-sensitivity differential scanning calorimeter (DSC3100SA from Bruker AXS), the melting point and the glass transition point were measured for each of the compounds having a benzotriazole ring structure synthesized in Examples 1 to 13. The measurement results are collectively shown in Table 1.

**[Table 1]**

| | Melting point | Glass transition point |
|---|---|---|
| Compound of Example 1 | 311°C | 151°C |
| Compound of Example 2 | 307°C | - |
| Compound of Example 3 | 326°C | - |
| Compound of Example 4 | 306°C | 139°C |
| Compound of Example 5 | 257°C | 117°C |
| Compound of Example 6 | 303°C | 137°C |
| Compound of Example 7 | 336°C | - |
| Compound of Example 8 | 312°C | 167°C |
| Compound of Example 9 | 302°C | 118°C |
| Compound of Example 10 | - | 121°C |
| Compound of Example 11 | - | 121°C |
| Compound of Example 12 | 329°C | 141°C |
| Compound of Example 13 | 283°C | 110°C |

The results show that the compounds having a benzotriazole ring structure synthesized in Examples 1 to 13 each have a glass transition point of 98°C or higher, thus indicating that they are stable in a thin-film state.

### Example 15:

A 100-nm-thick vapor deposition film was formed on an ITO substrate by using each of the compounds having a benzotriazole ring structure synthesized respectively in Examples 1 to 13, and the work function was measured using an ionization potential measurement device (PYS-202 from Sumitomo Heavy Industries, Ltd.). The measurement results are collectively shown in Table 2.

**[Table 2]**

| | Work function |
|---|---|
| Compound of Example 1 | 6.41 eV |
| Compound of Example 2 | 6.51 eV |
| Compound of Example 3 | 6.42 eV |
| Compound of Example 4 | 6.44 eV |
| Compound of Example 5 | 6.41 eV |
| Compound of Example 6 | 6.51 eV |
| Compound of Example 7 | 6.34 eV |
| Compound of Example 8 | 6.41 eV |
| Compound of Example 9 | 6.40 eV |
| Compound of Example 10 | 6.46 eV |
| Compound of Example 11 | 6.42 eV |
| Compound of Example 12 | 6.43 eV |
| Compound of Example 13 | 6.62 eV |

The results show that the compounds having a benzotriazole ring structure synthesized in Examples 1 to 13 have a greater work function than 5.5 eV, the work function of typical hole-transporting materials such as NPD, TPD, etc., and thus have high hole blocking capability.

### Example 16:

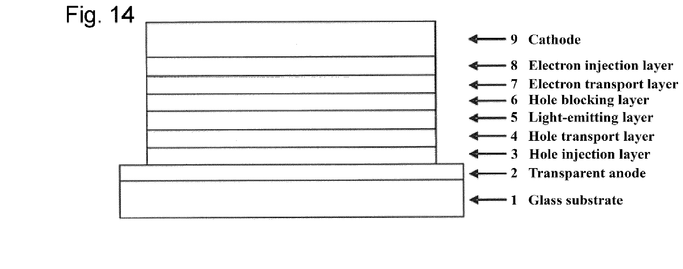

As illustrated in Fig. 14, an organic EL device was prepared by vapor-depositing a hole injection layer 3, a hole transport layer 4, a light-emitting layer 5, a hole blocking layer 6, an electron transport layer 7, an electron injection layer 8, and a cathode (aluminum electrode) 9 in this order onto a glass substrate 1 having formed thereon an ITO electrode as a transparent anode 2 in advance.

More specifically, a glass substrate 1 having formed thereon a 50-nm-thick ITO film as a transparent anode 2 was subjected to ultrasonic cleaning in isopropyl alcohol for 20 minutes, and then dried for 10 minutes on a hot plate heated to 200°C. Then, after UV ozone treatment for 15 minutes, the glass substrate having the ITO was mounted to a vacuum vapor deposition apparatus, in which the pressure was reduced to 0.001 Pa or lower. Next, a hole injection layer 3 was formed as the film of 10 nm thickness so as to cover the transparent anode 2, by performing binary vapor deposition of an electron acceptor (Acceptor-1) having the following structural formula and a compound (HTM-1) having the following structural formula at a rate at which the vapor deposition rate ratio between Acceptor-1 and HTM-1 was 3:97.

On this hole injection layer 3, the compound (HTM-1) having the following structural formula was formed as a hole transport layer 4 having a film thickness of 60 nm.

On this hole transport layer 4, a light-emitting layer 5 was formed so that the film thickness was 20 nm, by performing binary vapor deposition of a compound (EMD-1) having the following structural formula and a compound (EMH-1) having the following structural formula at a rate at which the vapor deposition rate ratio between EMD-1 and EMH-1 was 5:95.

On this light-emitting layer 5, a hole blocking layer-*cum-*electron transport layer 6, 7 was formed by performing binary vapor deposition of the compound of Example 1 (Compound-2) and a compound (ETM-1) having the following structural formula at a rate at which the vapor deposition rate ratio between Compound-2 and ETM-1 was 50:50, to the film thickness of 30nm.

On this hole blocking layer-*cum-*electron transport layer 6, 7, lithium fluoride was formed as an electron injection layer 8 having a film thickness of 1 nm.

Finally, on this electron injection layer 8, a cathode 9 was formed by vapor-depositing aluminum to a thickness of 100 nm.

Properties of the produced organic EL device were measured in the atmosphere at atmospheric temperature. Measurement results of light emission properties when a direct-current voltage was applied to the produced organic EL device are collectively shown in Table 3.

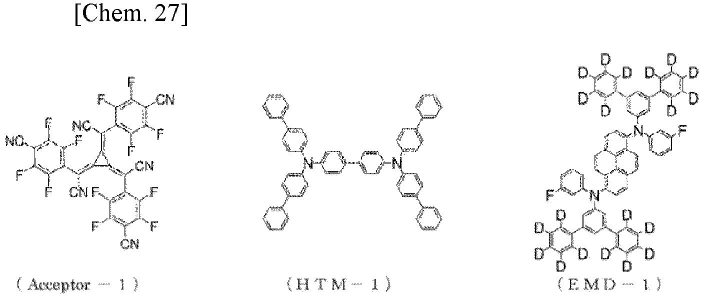

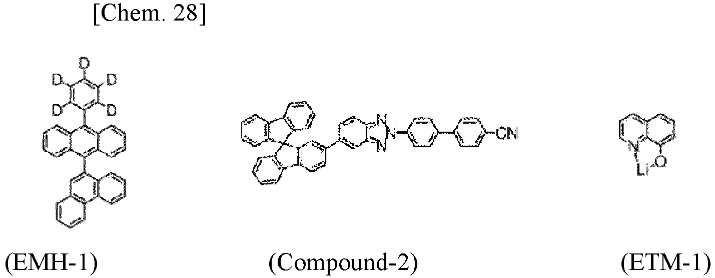

### Example 17:

An organic EL device was produced according to the same conditions in Example 16, except that, the compound (Compound-40) of Example 2 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-40 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 18:

An organic EL device was produced according to the same conditions in Example 16, except that, the compound (Compound-44) of Example 3 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-44 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 19:

An organic EL device was produced according to the same conditions in Example 16, except that, the compound (Compound-48) of Example 4 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-48 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 20:

An organic EL device was produced according to the same conditions in Example 16, except that, the compound (Compound-78) of Example 5 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-78 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 21:

An organic EL device was produced according to the same conditions in Example 16, except that, the compound (Compound-104) of Example 6 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-104 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 22:

An organic EL device was produced according to the same conditions in Example 16 , except that, the compound (Compound-141) of Example 7 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-141 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 23:

An organic EL device was produced according to the same conditions in Example 16, except that, the compound (Compound-155) of Example 8 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-155 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 24:

An organic EL device was produced according to the same conditions in Example 16, except that, the compound (Compound-164) of Example 9 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-164 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 25:

An organic EL device was produced according to the same conditions in Example 16, except that, the compound (Compound-172) of Example 10 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-172 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 26:

An organic EL device was produced according to the same conditions in Example 16, except that, the compound (Compound-184) of Example 11 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-184 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 27:

An organic EL device was produced according to the same conditions in Example 16 , except that, the compound (Compound-189) of Example 12 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-189 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

### Example 28:

An organic EL device was produced according to the same conditions in Example 16, except that, the compound (Compound-201) of Example 13 was used instead of the compound (Compound-2) of Example 1 as the material for the hole blocking layer-*cum-*electron transport layer 6, 7, and binary vapor deposition was performed at a vapor deposition rate at which the vapor deposition rate ratio between Compound-201 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

Comparative Example 1:
For comparison, an organic EL device was produced according to the same conditions in Example 16, except that, a compound (ETM-2) having the following structural formula (see, for example, Patent Literature 7) was used instead of the compound (Compound-2) of Example 1 as the material for the hole blocking layer-*cum-*electron transport layer 6, 7, and binary vapor deposition was performed at a vapor deposition rate at which the vapor deposition rate ratio between ETM-2 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

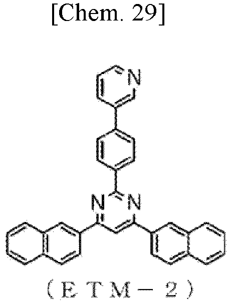

Comparative Example 2:
For comparison, an organic EL device was produced according to the same conditions in Example 16, except that, a compound (ETM-3) having the following structural formula (see, for example, Patent Literature 8) was used instead of the compound (Compound-2) of Example 1 as the material for the hole blocking layer-*cum-*electron transport layer 6, 7, and binary vapor deposition was performed at a vapor deposition rate at which the vapor deposition rate ratio between ETM-3 and ETM-1 was 50:50. Properties of the produced organic EL device were measured in the atmosphere at atmospheric temperature. Measurement results of light emission properties when a direct-current voltage was applied to the produced organic EL device are collectively shown in Table 3.

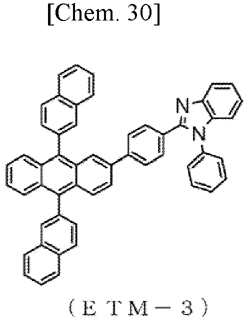

Device life was measured using the organic EL devices produced in Examples 16 to 28 and Comparative Examples 1 and 2. The results are collectively shown in Table 3. Device life was measured as following, constant current driving was performed with the light emission luminance at the start of light emission (i.e., initial luminance) being 2000 cd/m², and then the time for the light emission luminance attenuating to 1900 cd/m² (95% attenuation: the equivalent of 95% of the initial luminance, that considering to be 100%) was measured.

**[Table 3]**

| | Hole blocking layer-*cum*-electron transport layer | Voltage [V] (@10 mA/cm²) | Luminance [cd/m²] (@10 mA/cm²) | Luminous efficiency [cd/A] (@10 mA/cm²) | Power efficiency [lm/W] (@10 mA/cm²) | Device life, 95% attenuation |
|---|---|---|---|---|---|---|
| Example 16 | Compound-2/ ETM-1 | 3.51 | 883 | 8.84 | 7.93 | 365 hours |
| Example 17 | Compound-40/ ETM-1 | 3.67 | 863 | 8.63 | 7.40 | 305 hours |
| Example 18 | Compound-44/ ETM-1 | 3.68 | 884 | 8.84 | 7.56 | 374 hours |
| Example 19 | Compound-48/ ETM-1 | 3.71 | 883 | 8.85 | 7.51 | 286 hours |
| Example 20 | Compound-78/ ETM-1 | 3.58 | 905 | 9.05 | 7.95 | 326 hours |
| Example 21 | Compound-104/ ETM-1 | 3.58 | 897 | 8.97 | 7.88 | 262 hours |
| Example 22 | Compound-104/ ETM-1 | 3.50 | 877 | 8.78 | 7.89 | 263 hours |
| Example 23 | Compound-104/ ETM-1 | 3.54 | 887 | 8.87 | 7.89 | 373 hours |
| Example 24 | Compound-104/ ETM-1 | 3.52 | 870 | 8.70 | 7.77 | 279 hours |
| Example 25 | Compound-104/ ETM-1 | 3.55 | 909 | 9.10 | 8.05 | 389 hours |
| Example 26 | Compound-104/ ETM-1 | 3.43 | 880 | 8.81 | 8.08 | 385 hours |
| Example 27 | Compound-104/ ETM-1 | 3.50 | 868 | 8.69 | 7.82 | 344 hours |
| Example 28 | Compound-104/ ETM-1 | 3.55 | 834 | 8.34 | 7.39 | 405 hours |
| Comparative Example 1 | ETM-2/ETM-1 | 3.82 | 805 | 8.05 | 6.62 | 165 hours |
| Comparative Example 2 | ETM-3/ETM-1 | 4.01 | 659 | 6.59 | 5.16 | 203 hours |

As shown in Table 3, when a current having a current density of 10 mA/cm² was passed, the drive voltage was 3.82 to 4.01 V for the organic EL devices of Comparative Examples 1 and 2 using compounds ETM-2 and ETM-3 having the structural formulas shown above, whereas the organic EL devices of Examples 16 to 28 had a reduced voltage of 3.43 to 3.71 V. Further, while the organic EL devices of Comparative Examples 1 and 2 had a luminous efficiency of 6.59 to 8.05 cd/A, the organic EL devices of Examples 16 to 28 had improved luminous efficiency of 8.34 to 9.10 cd/A. Furthermore, while the organic EL devices of Comparative Examples 1 and 2 had a power efficiency of 5.16 to 6.62 lm/W, the organic EL devices of Examples 16 to 28 had significantly improved power efficiency of 7.39 to 8.08 lm/W. Particularly, while the organic EL devices of Comparative Examples 1 and 2 had a device life (95% attenuation) of 165 to 203 hours, the organic EL devices of Examples 16 to 28 had a significantly prolonged lifetime of 262 to 405 hours.

As described above, the organic EL devices according to the present invention excelled in luminous efficiency and power efficiency, compared to devices using compounds ETM-2 and ETM-3 having the structural formulas shown above, and it could achieve organic EL devices with a long lifetime.

### Industrial Applicability

Compounds having a specific benzotriazole ring structure according to the present invention have good electron injection capability and excellent hole blocking capability and are stable in a thin film state and are thus excellent for organic EL devices. By producing organic EL devices using these compounds, high efficiency can be achieved, driving voltage can be reduced, and durability can be improved also. For example, application can be expanded to home electrical appliances and lightings.

## Claims

1. A compound having a benzotriazole ring structure and being represented by general formula (a-1) as below: 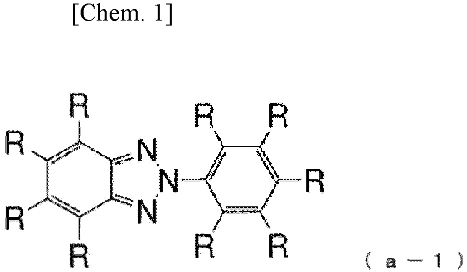
(in the formula, R may be the same or different from one another, and each represents a group represented by structural formula (b-1) as below, a hydrogen atom, a deuterium atom, a fluorine atom, a chlorine atom, a cyano group, a nitro group, a trimethylsilyl group, a triphenylsilyl group, a diphenylphosphinyl group, a diphenylphosphine oxide group, a substituted or unsubstituted aromatic hydrocarbon group, a substituted or unsubstituted aromatic heterocyclic group, a substituted or unsubstituted fused polycyclic aromatic group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, or a cycloalkyloxy group having 5 to 10 carbon atoms and optionally having a substituent, wherein
at least one of Rs is a group represented by the following structural formula (b-1)); 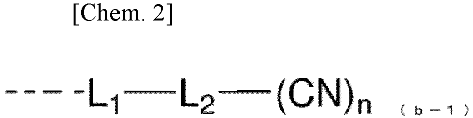
(in the formula, the broken line represents a bonding site,
L₁ represents a single bond, a substituted or unsubstituted aromatic hydrocarbon group, a substituted or unsubstituted aromatic heterocyclic group, or a substituted or unsubstituted fused polycyclic aromatic group,
L₂ represents a substituted or unsubstituted aromatic hydrocarbon group, a substituted or unsubstituted aromatic heterocyclic group, or a substituted or unsubstituted fused polycyclic aromatic group, and
n is an integer of 1 or 2, wherein
L₂ is a trivalent group when n is the integer 2).

2. The compound having a benzotriazole ring structure according to claim 1, represented by general formula (a-2) as below: 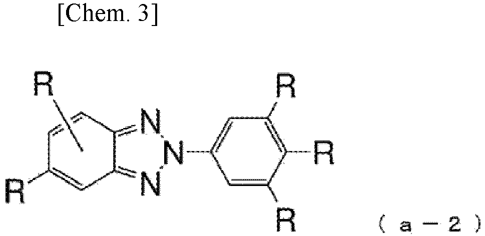 (in the formula, R has the same meaning as in the general formula (a-1)).

3. The compound having a benzotriazole ring structure according to claim 2, represented by general formula (a-3) as below: 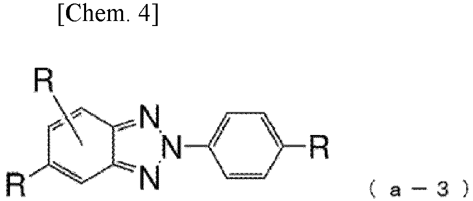 (in the formula, R has the same meaning as in the general formula (a-1)).

4. The compound having a benzotriazole ring structure according to claim 2, represented by general formula (a-4) as below: 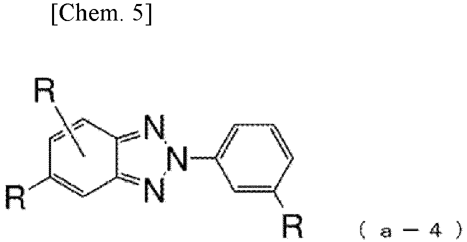 (in the formula, R has the same meaning as in the general formula (a-1)).

5. The compound having a benzotriazole ring structure according to claim 2, represented by general formula (a-5) as below: 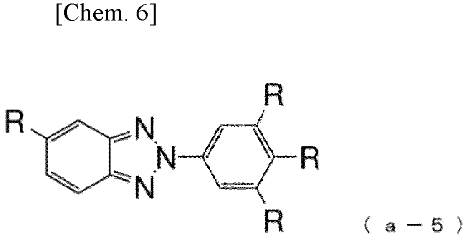 (in the formula, R has the same meaning as in the general formula (a-1)).

6. The compound having a benzotriazole ring structure according to claim 5, represented by general formula (a-6) as below: 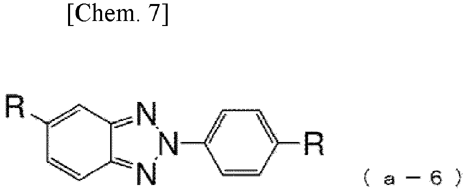 (in the formula, R has the same meaning as in the general formula (a-1)).

7. The compound having a benzotriazole ring structure according to claim 5, represented by general formula (a-7) as below: 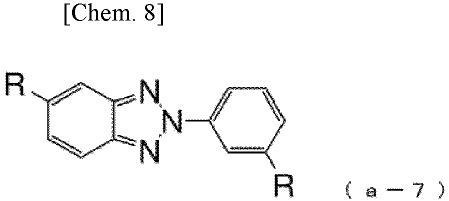 (in the formula, R has the same meaning as in the general formula (a-1)).

8. The compound having a benzotriazole ring structure according to any one of claims 1 to 7, wherein n in the structural formula (b-1) is the integer 1.

9. The compound having a benzotriazole ring structure according to any one of claims 1 to 7, wherein L₂ in the structural formula (b-1) is a substituted or unsubstituted aromatic hydrocarbon group.

10. An organic electroluminescence device comprising:
a pair of electrodes; and
at least one organic layer sandwiched between the electrodes, wherein
the at least one organic layer is an organic layer containing the compound having a benzotriazole ring structure according to any one of claims 1 to 9.

11. The organic electroluminescence device according to claim 10, wherein the organic layer containing the compound having a benzotriazole ring structure is an electron transport layer.

12. The organic electroluminescence device according to claim 10, wherein the organic layer containing the compound having a benzotriazole ring structure is a hole blocking layer.

13. The organic electroluminescence device according to claim 10, wherein the organic layer containing the compound having a benzotriazole ring structure is a light-emitting layer.

14. The organic electroluminescence device according to claim 10, wherein the organic layer containing the compound having a benzotriazole ring structure is an electron injection layer.
